# EUROPEAN PATENT SPECIFICATION

(11) **EP 0 737 672 B1**
(45) Date of publication and mention of the grant of the patent: **04.10.2001**
(21) Application number: 96105485.5
(22) Date of filing: 04.04.1996
(51) Int. Cl.: C07C 281/14, C07C 281/10, C07D 253/06, C07D 401/10, A01N 43/707, A61K 31/53, C07D 277/32, C07D 271/06, C07D 213/61, C07D 333/28

(54) **Method of producing triazine derivatives**
Verfahren zur Herstellung von Triazinderivate
Procédé de préparation de dérivés de triazine

(30) Priority: 14.04.1995 JP 8978695
(43) Date of publication of application: 16.10.1996
(73) Proprietor: TAKEDA SCHERING-PLOUGH ANIMAL HEALTH K.K., Osaka 541-0046 (JP)
(72) Inventor: Miki, Hideki, Toyono-gun, Osaka 563-01 (JP); Iwanaga, Koichi, Ikeda, Osaka 563 (JP); Aoki, Isao, Kawanishi, Hyogo 666-01 (JP)
(74) Representative: Winter, Brandl, Fürniss, Hübner, Röss, Kaiser, Polte Partnerschaft

(56) References cited:
- EP-A- 0 232 932
- EP-A- 0 476 439
- EP-A- 0 648 760
- WO-A-86/00072
- JOURNAL OF MEDICINAL CHEMISTRY, vol. 15, no. 3, 1972, pages 270-273, XP000575894 D.L.PUGH ET AL: "Metabolism of 1-[(5-nitrofurfurylidene)amino]-2-imidazol idinone"

## Description

### BACKGROUND OF THE INVENTION

### 1. Field of the Invention

This invention relates to a novel method of producing triazine derivatives, and also relates to a novel semicarbazone derivative which can be employed in the method of this invention. This invention also relates to a novel triazine derivative.

### 2. Description of the Prior Art

A 1,2,4-triazine derivative having a substituent at the 2-position has been widely used as an agent for controlling pests in the fields of medicines, veterinary drugs and agricultural drugs. For example, as veterinary drugs, a report is found in JPA H2(1990)-240003 that the compound is employable for controlling parasitic worms in fish and insects, and, another report is found in JPA H5(1993)-1047 that the compound is effective for controlling parasitic protozoa, especially coccidia. And, as agricultural chemicals, the effectiveness of the compound as a herbicide is reported in WO-A-86/0072 (Jan.3, 1986; FMC Co.).

As described thus above, 1,2,4-triazine derivatives having a substituent at 2-position, especially -3,5-dione derivatives and -3-one derivatives, are remarkably useful compounds for controlling pests. The present inventors considered that finding of a method of producing these compounds simply and conveniently would make a contribution to social welfare, and started the present research work.

As methods of synthesizing a 1,2,4-triazine-3,5-dione derivative having a substituent at the 2-position, a method which comprises allowing a hydrazone derivative to react with a keto-carboxylic acid [WO-A-86/00072] and a method which comprises allowing an active methylene compound (e.g. cyanoacetylurethane) to react with diazonium salt then subjecting the reaction mixture to ring-closure, decarboxylation reaction to produce the object compound [Journal of Medicinal Chemistry, Vol.22, p.1483, 1977] have been known. These methods, however, are accompanied with such drawbacks as having a relatively large number of reaction steps and requiring relatively drastic reaction conditions, thus being difficult to conduct on an industrial scale.

Especially no practical method of producing a 1,2,4-triazin-3-one derivative having a substituent at the 2-position has been known.

### SUMMARY OF THE INVENTION

The present invention is to provide a method of producing simply and conveniently triazine derivatives in a high yield which can be used as medicines, veterinary drugs and agricultural chemicals.

In view of the above-described technical background, the present inventors conducted extensively various studies for establishing a method of producing, for example, a 2-substituted-1,2,4-triazin-3-one derivative in which the 5-position of the triazine ring is unsubstituted. As a result, we found that a 2-substituted-1,2,4-triazine-3,5-dione derivative can be produced in a high yield by allowing a hydrazone derivative represented by the general formula (I) as defined hereinafter, to react with 2,2-dialkoxyethyl isocyanate in the presence of a base, then subjecting the reaction mixture to ring-closure reaction to produce a 2-substituted-1,2,4-triazin-3-one derivative (III), followed by subjecting thus-produced 2-substituted-1,2,4-triazine-3,5-dione derivative to oxidation. They have conducted further studies diligently to accomplish the present invention. Accordingly, the present invention provides a method of producing a 1,2,4-triazine-3-one derivative represented by the chemical formula: 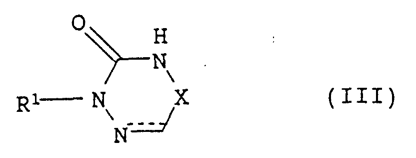 wherein R¹ stands for a C₆₋₁₄ aryl group or a 5- to 6-membered aromatic heterocyclic group, wherein each group may optionally have one to five substituents selected from the group consisting of (1) C₁₋₄ alkyl groups, (2) C₂₋₄ alkenyl groups, (3) C₇₋₁₁ aralkyl groups, (4) phenyl group, (5) C₁₋₆ alkoxy groups, (6) phenoxy group, (7) C₁₋₆ alkanoyl groups, (8) benzoyl group, (9) carboxyl group, (10) C₂₋₇ alkoxycarbonyl groups, (11) carbamoyl group, (12) N-mono-C₁₋₄ alkylcarbamoyl groups, (13) N,N-di-C₁₋₄ alkylcarbamoyl groups, (14) halogen atoms, (15) mono-, di- or tri-halogeno-C₁₋₄ alkyl groups, (16) optionally protected amino groups, (17) mono-C₁₋₄ alkylamino groups, (18) C₁₋₆ alkanoylamino groups, (19) benzoylamino groups, (20) carbamoylamino group, (21) N-C₁₋₄ alkylcarbamoylamino groups, (22) N,N-di-C₁₋₄ alkylcarbamoylamino groups, (23) C₁₋₃ alkylenedioxy groups, (24) hydroxy group, (25) nitro group, (26) cyano group, (27) mercapto group, (28) sulfo group, (29) sulfino group, (30) phosphono group, (31) sulfamoyl group, (32) C₁₋₆ monoalkylsulfamoyl groups, (33) di-C₁₋₄ alkylsulfamoyl groups, (34) C₁₋₆ alkylthio groups, (35) phenylthio group, (36) C₁₋₆ alkylsulfinyl groups, (37) phenylsulfinyl group, (38) C₁₋₆ alkylsulfonyl groups, (39) phenylsulfonyl group and (40) a 5- or 6-membered heterocyclic group containing 1 to 4 hetero-atoms selected from oxygen atom, sulfur atom and nitrogen atom, which may optionally be linked to the above-mentioned C₆₋₁₄ aryl group or 5- to 6-membered aromatic heterocyclic group through an atomic chain consisting of 1 to 4 carbon atoms, oxygen atoms and nitrogen atoms, further above-mentioned groups having carbon chain or cyclic group containing two or more carbon atoms may optionally have one to four substituents selected from the group consisting of (a) halogen atoms, (b) hydroxy group, (c) C₁₋₄ alkoxy groups or oxo group, (d) di-C₁₋₄ alkylamino groups, (e) halogeno-C₁₋₄ alkyl groups, (f) C₁₋₇ acyl groups, (g) hydroxy-C₁₋₄ alkyl groups, (h) C₁₋₄ alkoxy-C₁₋₄ alkyl groups,
(i) sulfamoyl and C₁₋₅ sulfamoyl groups,
(j) carbamoyl and C₁₋₇ carbamoyl group, (k) C₂₋₄ alkyl groups, (1) carboxyl group, (m) C₁₋₇ alkoxycarbonyl group and phenoxycarbonyl;
   X stands for carbonyl group, thiocarbonyl group or methylene group; and dashed line means that a double bond may optionally be formed; which comprises
(i) allowing a hydrazone derivative represented by the chemical formula: 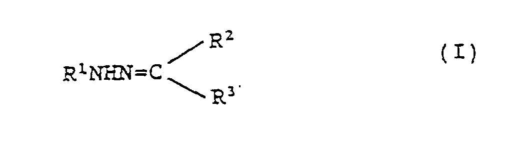 wherein R¹ has the same meaning as defined above; and R² and R³ stand for (1) hydrogen, (2) hydrocarbon residual group selected from the group consisting of C₁₋₄ alkyl groups, aromatic homocyclic groups and 5- or 6-membered aromatic heterocyclic groups, or (3) an electron withdrawing group selected from the group consisting of cyano group, C₁₋₆ alkoxy-carbonyl, hydroxycarbonyl, C₆₋₁₀ aryloxy-carbonyl groups, a 5- or 6-membered heterocyclic-oxycarbonyl group containing 1 to 4 hetero-atoms selected from nitrogen atom, sulfur atom and oxygen atom, C₁₋₆ alkylsulfonyl groups which may optionally be substituted with one to three halogen atoms, aminosulfonyl, di-C₁₋₄ alkoxyphosphoryl groups, C₁₋₆ acyl groups which may optionally be substituted with one to three halogen atoms, carbamoyl and C₁₋₆ alkylsulfonylthiocarbamoyl groups, to react with dialkoxyethyl isocyanate represented by (R⁴O)₂CHCH₂NCO wherein R⁴ stands for a C₁₋₄ alkyl group or one R⁴ and the other R⁴ may be optionally combine with each other to form a ring with an alkylene chain, to give the semicarbazone derivative represented by the chemical formula: 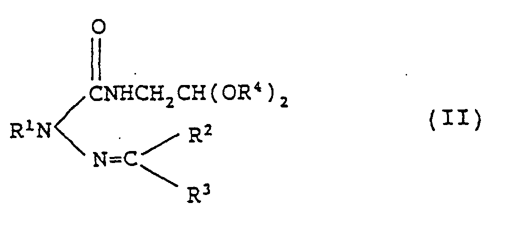 wherein respective groups have the same meanings as defined above and then
(ii)subjecting the semicarbazone derivative to a ring-closure reaction.

Examples of the optionally substituted aromatic homocyclic group include C₆₋₁₄ aryl groups such as phenyl, 1- or 2-naphthyl. Among them, preferable one is a phenyl group. Particularly preferable examples are a phenyl substituted at 3- and 4-positions and a phenyl substituted at 3-, 4- and 5-positions.

Examples of the 5- to 6-membered aromatic heterocyclic groups include an unsaturated 5- to 6-membered cyclic group containing, besides carbon atoms, 1 to 4 hetero-atoms such as oxygen atom, sulfur atom and nitrogen atom, such as a 5-membered cyclic group containing, besides carbon atoms, 1 to 4 hetero-atoms selected from oxygen atom, sulfur atom and nitrogen atom, e.g. 2- or 3-thienyl, 2- or 3-furyl, 2- or 3-pyrrolyl, 2-, 4- or 5-oxazolyl, 2-, 4- or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl and 1H- or 2H-tetrazolyl, and, a 6-membered cyclic group containing, besides carbon atoms, 1 to 4 hetero-atoms selected from oxygen atom, sulfur atom and nitrogen atom, e.g. 2-, 3- or 4-pyridyl, N-oxido-2, 3- or 4-pyridyl, 2-, 4- or 5-pyrimidinyl, N-oxido-2-, 4- or 5-pyrimidinyl, dioxotriazinyl, pyranyl, thiopyranyl, 1,3-oxazinyl, 1,4-thiazinyl, 1,3-thiazinyl, triazinyl, oxotriazinyl, 3- or 4-pyridazinyl, pyrazinyl and N-oxido-3- or 4-pyridazinyl. Among them, a 6-membered ring containing one heteroatom is preferable, and a N-containing hetero-ring, for example, is especially preferable.

Such aromatic homocyclic or heterocyclic groups as above may optionally have, at any possible position, 1 to 5, preferably 1 to 3 substituents selected from, for example,
(1) C₁₋₄ alkyl groups such as methyl, ethyl, propyl, isopropyl, butyl, isobutyl, sec-butyl and tert-butyl,
(2) C₂₋₄ alkenyl groups such as vinyl, 1-methylvinyl, 1-propenyl and allyl,
(3) C₇₋₁₁ aralkyl groups such as benzyl, α-methylbenzyl, α-cyanobenzyl, α-hydroxybenzyl and phenethyl,
(4) phenyl group,
(5) C₁₋₆ alkoxy groups such as methoxy, ethoxy, propoxy, iso-propoxy, n-butoxy, iso-butoxy, sec-butoxy and tert-butoxy group,
(6) phenoxy group,
(7) C₁₋₆ alkanoyl groups such as formyl, acetyl, propionyl, n-butyryl and iso-butyryl group,
(8) benzoyl group,
(9) carboxyl group,
(10) C₂₋₇ alkoxycarbonyl groups such as methoxycarbonyl, ethoxycarbonyl, n-propoxycarbonyl, iso-propoxycarbonyl, n-butoxycarbonyl, isobutoxycarbonyl and tert-butoxycarbonyl group,
(11) carbamoyl group,
(12) N-mono-C₁₋₄ alkylcarbamoyl groups such as N-methylcarbamoyl, N-ethylcarbamoyl, N-propylcarbamoyl, N-isopropylcarbamoyl and N-butylcarbamoyl,
(13) N,N-di-C₁₋₄ alkylcarbamoyl groups such as N,N-dimethylcarbamoyl, N,N-diethylcarbamoyl, N,N-dipropylcarbamoyl and N,N-dibutylcarbamoyl,
(14) halogen atoms such as fluorine, chlorine, bromine and iodine,
(15) mono-, di- or tri-halogeno-C₁₋₄ alkyl groups such as chloromethyl dichloromethyl, trifluoromethyl and trifluoroethyl,
(16) optionally protected amino groups,
(17) mono-C₁₋₄ alkylamino groups such as methylamino, ethylamino, propylamino, isopropylamino and butylamino,
(18) C₁₋₆ alkanoylamino groups such as formamido, acetamino, trifluoroacetamido, propionamido, butylamido and isobutylamido,
(19) benzoylamino groups such as benzamido,
(20) carbamoylamino group,
(21) N-C₁₋₄ alkyl carbamoylamino groups such as N-methyl carbamoylamino, N-ethyl carbamoylamino, N-propyl carbamoylamino, N-isopropyl carbamoylamino and N-butyl carbamoylamino,
(22) N,N-di-C₁₋₄ alkyl carbamoylamino groups such as N,N-dimethyl carbamoylamino, N,N-diethyl carbamoylamino, N,N-dipropyl carbamoylamino and N,N-dibutyl carbamoylamino,
(23) C₁₋₃ alkylenedioxy groups such as methylenedioxy and ethylenedioxy group,
(24) hydroxy group,
(25) nitro group,
(26) cyano group,
(27) mercapto group,
(28) sulfo group,
(29) sulfino group,
(30) phosphono group,
(31) sulfamoyl group,
(32) C₁₋₆ monoalkyl sulfamoyl groups such as N-methyl sulfamoyl, N-ethyl sulfamoyl, N-propyl sulfamoyl, N-isopropyl sulfamoyl and N-butyl sulfamoyl,
(33) di-C₁₋₄ alkyl sulfamoyl groups such as N,N-dimethyl sulfamoyl, N,N-diethyl sulfamoyl, N,N-dipropyl sulfamoyl and N,N-dibutyl sulfamoyl,
(34) C₁₋₆ alkylthio groups such as methylthio, ethylthio, propylthio, isopropylthio, n-butylthio, sec-butylthio and tert-butylthio group,
(35) phenylthio group,
(36) C₁₋₆ alkyl sulfinyl groups such as methyl sulfinyl, ethyl sulfinyl, propyl sulfinyl and butyl sulfinyl,
(37) phenyl sulfinyl group,
(38) C₁₋₆ alkyl sulfonyl groups such as methyl sulfonyl, ethyl sulfonyl, propyl sulfonyl and butyl sulfonyl group,
(39) phenyl sulfonyl group, and
(40) a 5- or 6-membered heterocyclic group containing, besides carbon atoms, 1 to 4 hetero-atoms selected from oxygen atom, sulfur atom and nitrogen atom, which may optionally be linked to the above-mentioned aromatic cyclic group through 1 to 4 carbon atoms, oxygen atoms, nitrogen atoms and atomic chain consisting of, for example, oxygen atom, such as 2- or 3-thienyl, 2- or 3-furyl, 2- or 3-pyrrolyl, 2-, 3- or 4-pyridyl, 2-, 4- or 5-oxazolyl, 2-, 4- or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl, 1H- or 2H-tetrazolyl, N-oxido-2-, 3-or 4-pyridyl, 2-, 4- or 5-pyrimidinyl, N-oxido-2-, 4-or 5-pyrimidinyl, oxoimidazinyl, dioxotriazinyl, pyranyl, thiopyranyl, 1,4-oxazinyl, 1,4-thiazinyl, 1,3-thiazinyl, triazinyl, oxotriazinyl, 3- or 4-pyridazinyl, pyrazinyl and N-oxido-3- or 4-pyridazinyl.

Among the above-mentioned hydrocarbon residual groups shown by R¹, optionally substituted phenyl groups are preferable. Especially preferable examples of such substituents include an optionally substituted C₇₋₁₁ aralkyl groups (3) and/or halogen atom (14). The phenyl having substituents at 3- or/and 5-positions and 4-position, and the phenyl having substituents at 3- and/or 5-position and 4-position are especially preferable.

Among these groups, those having carbon chain or cyclic group containing two or more carbon atoms may optionally have, at any possible position, further one to four, preferably one or two substituents selected from, for example,
(a) halogen atoms such as chlorine, fluorine, bromine and iodine,
(b) hydroxy group,
(c) C₁₋₄ alkoxy groups such as methoxy and ethoxy, or oxo group,
(d) di-C₁₋₄ alkylamino groups such as dimethylamino and diethylamino,
(e) halogeno-C₁₋₄ alkyl groups such as chloromethyl, 1-chloroethyl, 1-fluoroethyl, fluoromethyl, trifluoromethyl and trifluoroethyl,
(f) C₁₋₇ acyl groups such as formyl, acetyl, propionyl, isopropionyl, trifluoroacetyl and benzoyl,
(g) hydroxy-C₁₋₄ alkyl groups such as hydroxymethyl, 1-hydroxyethyl and 2-hydroxyethyl,
(h) C₁₋₄ alkoxy-C₁₋₄ alkyl groups such as methoxymethyl, 1-methoxyethyl, 1-ethoxyethyl, acetoxyethyl and 2-ethoxyethyl,
(i) C₁₋₅ sulfamoyl groups such as aminosulfonyl, methylaminosulfonyl, dimethylaminosulfonyl and morpholynosulfonyl,
(j) C₁₋₇ carbamoyl group such as aminocarbonyl, methylaminocarbonyl, dimethylaminocarbonyl and phenylaminocarbonyl,
(k) C₂₋₄ alkyl groups such as ethyl and isopropyl,
(l) carboxyl group,
(m) C₁₋₇ alkoxycarbonyl group such as methoxycarbonyl, ethoxycarbonyl and phenoxycarbonyl.

For example, in the case that the C₇₋₁₁ aralkyl group (3) is benzyl, the benzyl substituted at its 4-position with the substituent (f) or (g) is preferable.

In the above formulae, as the optionally substituted hydrocarbon residual groups shown by R² or R³, mention is made of, for example, optionally substituted alkyl groups and optionally substituted aromatic homocyclic groups or 5- to 6-membered aromatic heterocyclic groups.

Among them, as the alkyl group, use is preferably made of C₁₋₄ lower alkyl groups (e.g. methyl), as the aromatic homocyclic group, use is preferably made of phenyl group, and, as the aromatic heterocyclic groups, use is preferably made of 2-, 3- or 4-pyridyl group.

Examples of the electron withdrawing groups shown by R² or R³ include cyano group, C₁₋₆ alkoxy-carbonyl such as methoxy carbonyl and ethoxy carbonyl, hydroxy carbonyl, C₆₋₁₀ aryloxy carbonyl groups such as phenyloxy carbonyl and naphthyloxy carbonyl, a 5- or 6-membered heterocyclic-oxycarbonyl group containing, besides carbon atoms, 1 to 4 hetero-atoms selected from nitrogen atom, sulfur atom and oxygen atom, such as pyridyloxy carbonyl and thienyloxy carbonyl, C₁₋₆ alkyl sulfonyl groups optionally substituted with one to three halogen atoms selected from, for example, chlorine, bromine and fluorine, such as methyl sulfonyl, trifluoromethyl sulfonyl and ethyl sulfonyl, amino sulfonyl, di-C₁₋₄ alkoxy phosphoryl groups such as dimethoxyphosphoryl, diethoxyphosphoryl and dipropoxyphosphoryl, C₁₋₆ acyl groups optionally substituted with one to three halogen atoms selected from chlorine, bromine and fluorine, such as acetyl and propionyl, carbamoyl, and C₁₋₆ alkyl-sulfonyl thiocarbamoyl groups such as methyl sulfonyl thiocarbamoyl and ethyl sulfonyl thiocarbamoyl group.

As the preferable example of R² and R³, mention is made of hydrogen as one of R² and R³ and phenyl as the other.

Further, R² and R³ may optionally be combined with each other to form a 4- to 7-membered ring such as cycloalkyl groups.

Examples of the optionally substituted alkyl groups shown by R⁴ include C₁₋₄ alkyl groups, and especially methyl and ethyl are preferable. One R⁴ and the other R⁴ may be optionally conbined with each other to form a ring with an alkylene chain (e.g. (̵CH₂)̵₂).

In the production method of this invention, the intermediate compound, a semicarbazone derivative (II), is produced by the method as shown below.

The reaction step of producing the intermediate semicarbazone derivative (II) by allowing a hydrazone derivative (I) to react with 2,2-dialkoxyethyl isocyanate, which is shown by the following reaction: 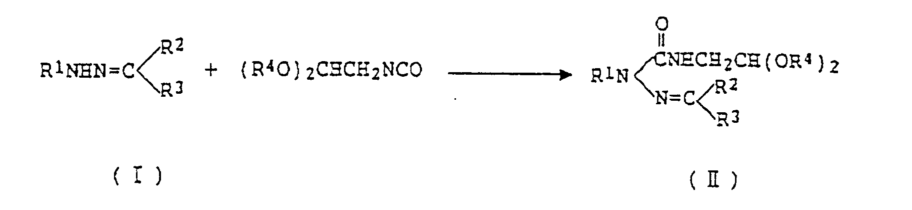 wherein R¹, R², R³ and R⁴ are of the same meaning as defined above, is conducted usually in an inert solvent or in the absence of solvent, optionally in the presence of a base. While the reaction temperature varies with the kinds of solvents employed, it ranges usually from about -20 to 110°C, especially preferably from about 0 to 50°C. While the reaction time varies with the kinds of solvents employed, it ranges usually from about 10 minutes to 5 hours, preferably from 30 minutes to 2 hours.

As the solvent for this reaction, almost all inert solvents can be employed, i.e. solvents commonly employed in the general chemical reactions, as exemplified by benzene, ligroin, benzine, toluene, xylene, methylene chloride, ethylene chloride, chloroform, carbon tetrachloride, dichloroethane, chlorobenzene, o-dichlorobenzene, ethers (e.g. diethyl ether, diisopropyl ether, dibutyl ether, glycol dimethyl ether, diglycol dimethyl ether, tetrahydrofuran and dioxane), ketone (e.g. acetone, methyl ethyl ketone, methyl isopropyl ketone and methyl isopropyl ketone), ester (e.g. ethyl acetate ester), nitrile (e.g. acetonitrile and propionitrile), amide (e.g. dimethylformamide, dimethylacetamide and hexamethyl phosphoric triamide), dimethyl sulfoxide and pyridine.

The amount of 2,2-dialkoxy ethyl isocyanate ranges usually from 1.0 to 3.5 mol., preferably from 1.0 to 1.5 mol., relative to the hydrazone derivative (I).

For allowing the reaction to proceed smoothly, a base may optionally be added. Examples of the base to be employed for this purpose include inorganic bases such as sodium carbonate, potassium carbonate, calcium carbonate, sodium hydroxide and potassium hydroxide, and organic bases such as triethylamine, pyridine, dimethyl aniline, picoline, 1,5-diazabicyclo[4,3,0]non-5-ene, 1,4-diazabicyclo[2,2,2]octane, 1,8-diazabicyclo[5,4,0]-7-undecene (DBU). The amount of the base to be employed ranges from 0.001 to 30.0%, preferably from 0.01 to 5.0%, relative to the starting material (I).

Among the semicarbazone derivative (II) obtained by the above method, the derivative (II') having a residual group: 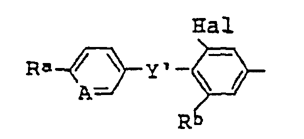 wherein R^{a} is acyl, sulfamoyl and C₁₋₅ sulfamoyl, carbamoyl and C₁₋₇ carbamoyl groups, carboxyl, alkoxycarbonyl, optionally substituted alkyl or optionally substituted amino; A is -CH- or nitrogen atom, Y' is methylene, cyanomethylene, carbonyl, hydroxymethylene, sulfur atom, sulfinyl, sulfonyl or oxygen atom; Hal is halogen (e.g. chlorine), R^{b} hydrogen, halogen (e.g . chlorine) or lower alkyl (e.g. methyl) as the symbol R¹ is a novel compound and important intermediate for obtaining useful 1,2,4-triazine-3-one derivative (III).

The semicarbazone derivative (II) synthesized by the above-described reaction is subjected to a ring-closure reaction by a conventional method to convert into a 2-substituted-1,2,4-triazin-3-one derivative (III).

The derivative (III) includes the derivatives (III-a), (III-b), (III-c), (III-d) and (III-e). Each derivative can be produced by the following steps.

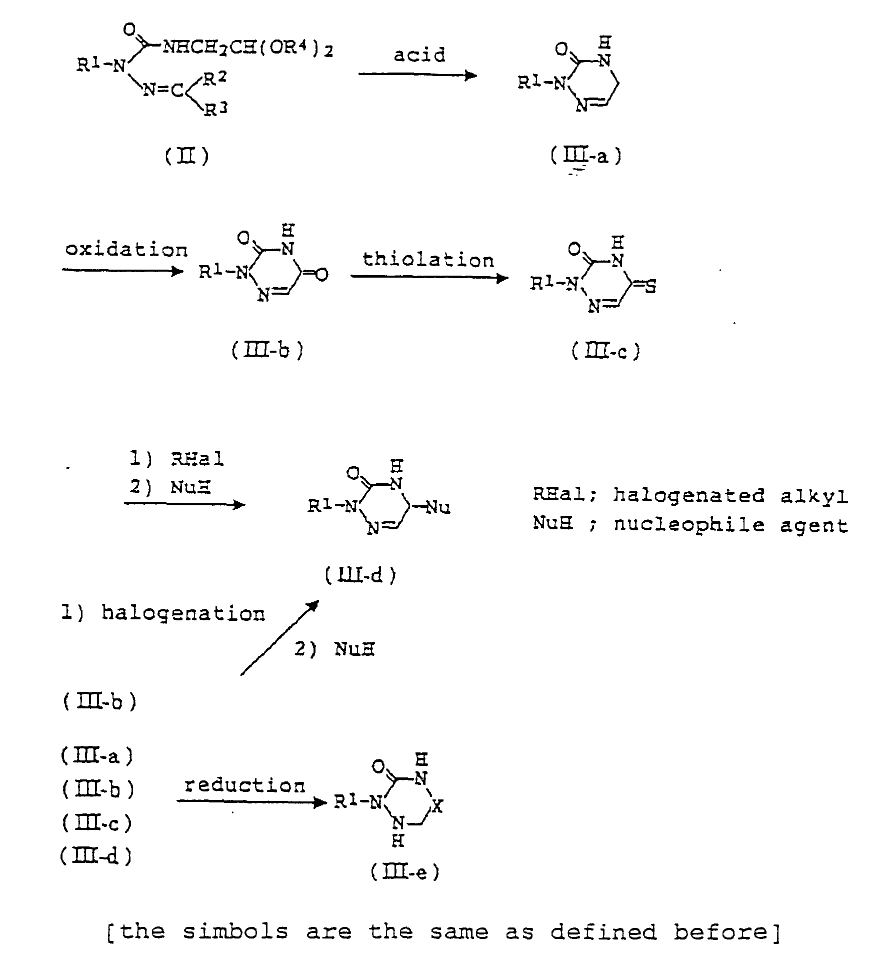

The 2-substituted-1,2,4-triazine-3-one derivative (III-a) is produced by ring-closure reaction of the semicarbazone derivative (II) and the 2-substituted-1,2,4-triazine-3,5-dione derivative (III-b) is produced by oxidation reaction of the derivative (III-a). The 2-substituted-1,2,4-triazine-3-one-5-thione derivative (III-c) is obtainable by thiolation reaction of the derivative (III-b). The 2-substituted-5-mono (or di) substituted-1,2,4-triazine-3-one (III-d) is producible from the derivative (III-c) or (III-b). Thus obtained derivative (III-a), (III-b), (III-c) or (III-d) is subjected to reduction reaction to obtain the derivative (III-e) in high yield.

The reaction (II) → (II-a) is conducted usually in an inert solvent or in the absence of solvent, optionally in the presence of an acid. While the reaction temperature varies with the kinds of solvents employed for the reaction, it ranges usually from about -20 to 150°C, especially from about 0 to 80°C. While the reaction time varies with the kinds of solvents employed for the reaction, it ranges usually from about 10 minutes to 5 hours, preferably from 30 minutes to 2 hours.

As the solvent for this reaction, almost all inert solvents can be employed, i.e. solvents commonly employed in the general chemical reactions, as exemplified by benzene, ligroin, benzine, toluene, xylene, methylene chloride, ethylene chloride, chloroform, carbon tetrachloride, dichloroethane, chlorobenzene, o-dichlorobenzene, ethers (e.g. diethyl ether, diisopropyl ether, dibutyl ether, glycol dimethyl ether, diglycol dimethyl ether, tetrahydrofuran and dioxane), ketone (e.g. acetone, methyl ethyl ketone and methyl isopropyl ketone), ester (e.g. ethyl acetate ester), nitrile (e.g. acetonitrile and propionitrile), amide (e.g. dimethylformamide, dimethylacetamide and hexamethyl phosphoric acid triamide), alcohol (e.g. methyl alcohol, ethyl alcohol propyl alcohol, isopropyl alcohol), pyridine and dimethyl sulfoxide.

In the reaction of converting a semicarbazone derivative (II) to a 2-substituted-1,2,4-triazin-3-one derivative by ring-closure reaction, an acid or a Lewis acid may be added for the purpose of allowing the reaction to proceed smoothly. Examples of the acid employed for this purpose include trichloroacetic acid, trifluoroacetic acid, p-toluene sulfonic acid, methane sulfonic acid, sulfuric acid, hydrochloric acid, phosphoric acid and polyphosphoric acid and example of the Lewis acid is trifluoroborane etherate.

In the production method of this invention, the object 1,2,4-triazin-3-one derivative (III) can be produced in a high yield by subjecting the reaction mixture to ring-closure reaction, without isolating the semicarbazone derivative (II) from the reaction mixture obtained by the above-mentioned reaction a). This one series step (one-pot reaction) is preferably employed for producing the object compound (III) on an industrial scale.

The 1,2,4-triazin-3-one derivative (III-a) produced by the above-described reaction step can be used per se as agricultural chemicals and drugs for controlling parasitic pest. -By subjecting these compounds to further reaction such as oxidation, reduction and substitution in accordance with conventional methods, various triazine derivatives such as 1,2,4-triazine-3,5-dione derivatives, 1,2,4-triazine-3-oxo-5-thione derivatives, 1,2,4-triazin-3-one-5-amino derivative and hexahydro-1,2,4-triazin-3-one derivatives can be produced conveniently and in a high yield. For
example, by employing a 2-substituted-1,2,4-triazin-3-one derivative synthesized by the above-described reaction step, a 2-substituted 1,2,4-triazine-3,5-dione derivative can be produced in a high yield by a conventional oxidation reaction. This oxidation reaction can be conducted by using a oxidizing agent described in, for example, I & II of Shin Jikken Kagaku Koza Vol. 15 (compiled by The Chemical Society of Japan, Published by Maruzen Co., Ltd., 1976).

This reaction is conducted usually in an inert solvent, optionally in the presence of a base or an acid. While the reaction temperature varies with the kinds of solvents, it ranges usually from about 20 to 180°C, preferably from about 50 to 100°C. While the reaction time varies with the kinds of solvents, it ranges usually from about one hour to 15 hours, preferably from 3 hours to 8 hours. As the solvent for this reaction, almost all
inert solvents can be employed, i.e. solvents commonly employed in the general chemical reactions, as exemplified by benzene, ligroin, benzine, toluene, xylene, methylene chloride, ethylene chloride, chloroform, carbon tetrachloride, dichloroethane, chlorobenzene, o-dichlorobenzene, ethers (e.g. dibutyl ether, glycol dimethyl ether, diglycol dimethyl ether, tetrahydrofuran and dioxane), ketone (e.g. methyl ethyl ketone, methyl isopropyl ketone and methyl isopropyl ketone), ester (e.g. ethyl acetate ester), nitrile (e.g. acetonitrile and propionitrile), amide (e.g. dimethylformamide, dimethylacetamide and hexamethyl phosphoric acid triamide), dimethyl sulfoxide, mercaptoacetic acid, acetic acid and pyridine.

Among the 1,2,4-triazine derivative (III) which is produced by the method of the present invention, the following derivative (III') is very effective for controlling parasitic protozoa. 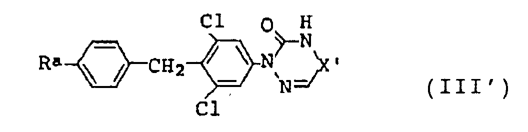 wherein R^{a} is C₁-C₇ acyl, a sulfamyol and C₁-C₅ sulfamoyl, a C₂-C₄ alkyl, halogeno- C₁-C₄ alkyl hydroxy, C₁-C₄ alkyl, or C₁-C₄ alkoxy-C₁-C₄ alkyl and X' is methylene or carbonyl.

The acyl means C₁₋₇ acyl such as formyl, acetyl, propionyl, isopropionyl, trifluoacetyl, and benzoyl. The sulfamoyl means C₁₋₅ sulfamoyl such as methylaminosulfonyl, dimethylaminosulfonyl and morpholinosulfonyl. The alkyl means C₂₋₄ alkyl such as ethyl and isopropyl, and halogeno-C₁₋₄ alkyl such as chloromethyl, 1-chloroethyl, 1-fluoroethyl, trifluoromethyl and trifluoroethyl, and hydroxy-C₁₋₄ alkyl such as hydroxymethyl, 1-hydroxyethyl and 2-hydroxyethyl, and C₁₋₄ alkoxy-C₁₋₄ alkyl such as methoxymethyl, 1-methoxyethyl, 1-ethoxyethyl, 1-acetoxyethyl and 2-ethoxyethyl.

Especially, 2-[4-(4-acetylbenzyl)-3,5-dichlorophenyl]-4,5-dihydro-1,2,4-triazine-3(2H)-one (Example No. 6) and 2-{3,5-dichloro-4-[4-(1-hydroxyethyl)benzyl]phenyl}-4,5-dihydro-1,2,4-triazine-3(2H)-one (Example No. 16) are very useful, because these show high effectiveness for controlling parasitic protozoa and have very low residue.

### WORKING EXAMPLE

### Reference Example 1

### Synthesis of α-(3,4-dichlorophenyl)-α-(2,6-dichloro-4-nitrophenyl)acetonitrile

To 150 ml of 20% hydrous DMSO were added 6.15 g of 3,4-dichlorobenzyl cyanide, 8.13 g of 4-bromo-3,5-dichloronitrobenzene and 1.50 g of sodium hydroxide. The reaction was allowed to proceed for one hour at temperatures ranging from 60 to 70°C. After completion of the reaction, DMSO was removed, and the residue was dissolved in 50 ml of toluene. The solution was washed with water, dried and concentrated. To the concentrate was added ethyl alcohol to cause crystallization to afford the titled compound in a yield of 67%, m.p.171-172°C
¹H-NMR(CDCl₃); 6.21(s,1H), 7.13-7.52(m,3H), 8.29(s,2H) Reference Example 2

### Synthesis of 3,5-dichloro-4-(3,4-dichloro-α-cyanobenzyl)aniline

In 100 ml of methanol were dissolved 7.6 g of α-(3,4-dichlorophenyl)-α-(2,6-dichloro-4-nitrophenyl)acetonitrile and 0.8 g (50%) of Raney's nickel. The solution was subjected to reduction with three times as much mol. of hydrogen gas. Insolubles were removed from the reaction mixture, then the remaining solution was concentrated to give the titled compound in a yield of 95%, m.p.191-194°C
¹H-NMR(CDCl₃); 3.97(br,2H), 5.98(s,1H), 6.67(s,2H), 7.12-7.47(m,3H)

### Reference Example 3

### Synthesis of 3,5-dichloro-4-(3,4-dichloro-α-cyanobenzyl)phenylhydrazine

In 40 ml of acetic acid was dissolved 3.0 g of 3,5-dichloro-4-(3,4-dichloro-α-cyanobenzyl)aniline. To the solution was then added 3 ml of 35% hydrochloric acid. To the mixture was added dropwise, while cooling at temperatures ranging from 10 to 12°C, a solution of 0.8 g of 98.5% sodium nitrite in 3 ml of water. The reaction mixture was stirred for 40 minutes under the same conditions, to which was then added 7.0 g of stannous chloride dissolved in 10 ml of 35% hydrochloric acid. The reaction mixture was poured into ice-water, which was then made into alkaline, followed by extraction with 200 ml of ethyl acetate. The extract was washed with water, dried over anhydrous magnesium sulfate, and concentrated to afford the titled compound in a yield of 96%, m.p.183-185°C.
¹H-NMR(DMSO-d₆); 4.26(br,2H), 6.25(s,1H), 6.87(s,2H), 7.11-7.72(m,4H)

### Reference Example 4

### Synthesis of 1-[3,5-dichloro-4-(3,4-dichloro-α-cyanobenzyl)phenyl]-2-benzylidenehydrazine.acetic acid ethyl ester

In 50 ml of acetic acid ethyl ester was dissolved 3.0 g of 3,5-dichloro-4-(3,4-dichloro-α-cyanobenzyl)phenylhydrazine. To the solution was added equimol. of benzaldehyde, and the mixture was stirred for 3 hours at room temperature. The reaction mixture was dried over anhydrous magnesium sulfate, which was then concentrated to afford the titled compound in a yield of 68%, m.p.75-90°C.
¹H-NMR(CDCl₃); 1.25(t,3H), 2.04(s,3H), 4.12(q,2H), 6.03(s,1H), 7.13(s,2H), 7.24-7.81(m,10H)

### Reference Example 5

### Synthesis of α-(4-chlorophenyl)-α-(2,6-dichloro-4-nitrophenyl)methane

To 50 ml of 20% hydrous DMSO were added 1.9 g of 4-chlorophenyl acetic acid methyl ester, 2.7 g of 4-bromo-3,5-dichloronitrobenzene and 0.5 g of sodium hydroxide. The reaction was allowed to proceed at temperatures ranging from 60 to 70°C for one hour, then at temperatures ranging from 130 to 135°C for 8 hours. After completion of the reaction, DMSO was removed. The residue was dissolved in 50 ml of toluene. The solution was washed with water, dried and concentrated. To the concentrate was added ethyl alcohol to cause crystallization to afford the titled compound in a yield of 91%, m.p.80-81°C.
¹H-NMR(CDCl₃); 4.36(s,2H), 7.17(q,4H), 8.20(s,2H)

### Reference Example 6

### Synthesis of α-(4-chlorophenyl)-α-(2,6-dichloro-4-aminophenyl)methane

In 30 ml of methanol were dissolved 1.6 g of α-(4-chlorophenyl)-α-(2,6-dichloro-4-nitrophenyl)methane and 0.2 g (50%) of Raney's nickel. The solution was subjected to reduction with three times as much mol. of hydrogen gas. Insolubles were removed and the remaining reaction mixture was concentrated to afford the titled compound in a yield of 95%, m.p.141-142°C.
¹H-NMR(CDCl₃); 3.72(br,2H), 4.14(s,2H), 6.65(s,2H), 6.95-7.44(m,4H)

### Reference Example 7

### Synthesis of α-(4-chlorophenyl)-α-(2,6-dichloro-4-hydrazinophenyl)methane

In 20 ml of acetic acid was dissolved 1.5 g of α-(4-chlorophenyl)-α-(2,6-dichloro-4-aminophenyl)methane. To the solution was then added 3 ml of 35% hydrochloric acid. To the mixture was added dropwise, while cooling at temperatures ranging from 10 to 12°C, 0.4 g of 98.5% sodium nitrite dissolved in 1 ml of water. The reaction mixture was stirred for 40 minutes under the same conditions, to which was then added 4.0 g of stannous chloride dissolved in 4 ml of 35% hydrochloric acid. The reaction mixture was poured into ice-water, and the pH of the solution was made alkaline, followed by extraction with 50 ml of acetic acid ethyl ester. The extract was washed with water, dried over anhydrous magnesium sulfate, and concentrated to afford the titled compound in a yield of 95%, m.p.129-130°C
¹H-NMR(CDCl₃); 3.57(br,2H), 4.17(s,2H), 5.23(br,1H), 6.83(s,2H), 7.03-7.27(q,4H)

### Reference Example 8

### Synthesis of α-(4-chlorophenyl)-α-(2,6-dichloro-4-benzylidene hydrazinophenyl)methane

In 50 ml of dichloromethane was dissolved 0.7 g of α-(4-chlorophenyl)-α-(2,6-dichloro-4-hydrazinophenyl)methane. To the solution was added equimol. of benzaldehyde, and the mixture was stirred for 3 hours at room temperature. The reaction mixture was dried over anhydrous magnesium sulfate, followed by concentration to afford the titled compound in a yield of 98%, m.p.149-150°C.
¹H-NMR(CDCl₃); 4.20(s,2H), 7.09-7.68(m,13H)

### Reference Example 9

### Synthesis of benzaldehyde 4-(4-chloro-α-cyanobenzyl)-3,5-dichlorophenyl hydrazone

In 30 ml of acetic acid ethyl ester was dissolved 3.26 g of 4-(4-chloro-α-cyanobenzyl)-3,5-dichlorophenyl hydrazine. To the solution was added equimolar benzaldehyde and three times as much mol. of anhydrous magnesium sulfate, then the reaction was allowed to proceed for one hour at room temperature. After completion of the reaction, insolubles were removed, and the solution was concentrated, which was recrystallized from acetonitrile to afford 3.3 g of the titled compound as colorless crystals, m.p.162-163°C.
¹H-NMR(CDCl₃); 6.05(s,1H), 7.09(s,2H), 7.30(s,4H), 7.30-8.00(m,7H)

### Reference Example 10

### Synthesis of 4-(4-Acetylbenzyl)-3,5-dichloroaniline

In 11 ml of ethylacetate were dissolved 1.1 g 4-(4-Acetylbenzyl)-3,5-dichloronitrobenzene and 3.8 g Tin (11) chloride dihydrate. The solution was allowed to proceed for one hour at temperatures ranging from 40-50°C. After completion of the reaction. The reaction mixture was poured into 100 ml ice-water, followed by extraction with 150 ml ethylacetate. The extract was washed with water and 25% ammonia solution, dried over anhydrous magnesium sulfate, and concentrated to afford the titled compound in a yield of 100%, m.p.97-98°C.
¹H-NMR(CDCl₃); 2.55(s,3H), 3.75(br,2H), 4.24(s,2H), 6.67(s,2H), 7.25(d,2H), 7.85(d,2H)

### Reference Example 11

### Synthesis of 2-[4-(4-Acetylbenzyl)-3,5-dichlorophenyl]-1-benzylidenehydrazine

In 10 ml of acetic acid was dissolved 1.0 g of 4-(4-Acetylbenzyl)-3,5-dichloroaniline. To the solution was then added 1.0 ml of 35% hydrochloric acid. To the mixture was added dropwise, while cooling at temperatures ranging from 8-10°C, a solution of 0.3 g of 98.5% sodium nitrite in 1.0 ml water. The reaction mixture was stirred for one hour under the same conditions, to which was then added 2.0 g of Tin (II) chloride dihydrate dissolved in 2.0 ml of 35% hydrochloric acid. The reaction mixture was stirred for 3 hours at room temperature. To the mixture then added 20 ml of water and 20 ml chloroform. To the mixture was added dropwise, while cooling at temperatures ranging from 5-10°C, 0.36 g of benzaldehyde. The reaction mixture was stirred for 30 minutes under the same conditions. After completion of the reaction, followed by extraction with 20 ml of chloroform. The extract was washed with water and sturated sodium hydrogen carbonate solution, dried over an hydrous magnesium sulfate, and concentrated to provide 0.88 g of the title compound as a colorless crystals, m.p.137-139°C.
¹H-NMR(CDCl₃); 2.56(s,3H), 4.29(s,2H), 7.11-7.90(m, 13H)

### Working Example 1

### Synthesis of 1-benzylidene-2-[4-(4-chlorobenzyl)-3,5-dichlorophenyl]-4-(2,2-diethoxyethyl)-semicarbazide

In 5 ml of acetonitrile was dissolved 0.5 g of α-(4-chlorophenyl)-α-(2,6-dichloro-4-benzylidene-hydrazinophenyl)methane. To the solution were added 0.3 g of 2,2-diethoxyethylisocyanate and 0.015 g of DBU. The mixture was stirred for one hour at room temperature. The reaction mixture was cooled, then the resulting crystalline precipitate was collected by filtration to give the titled compound in a yield of 97%, m.p.138-139°C,
¹H-NMR(CDCl₃); 1.27(t,6H), 3.46-3.90(m,6H), 4.33(s,2H), 6.92-7.63(m,13H)

### Working Example 2

### Synthesis of 1-benzylidene-2-[4-(4-chloro-α-cyanobenzyl)-3,5-dichlorophenyl]-4-(2,2-dimethoxyethyl)semicarbazide

In 20 ml of acetonitrile was suspended 3.2 g of benzaldehyde 4-(4-chloro-α-cyanobenzyl)-3,5-dichlorophenyl hydrazone. To the suspension were added 1.5 g of 2,2-dimethoxyethyl isocyanate and 20 mg of DBU. The reaction was allowed to proceed for one hour at room temperature, then the resulting crystalline precipitate was collected by filtration, which was washed with normal hexane, followed by drying to afford 3.7 g of the titled compound as colorless crystals, m.p.190-191°C.
¹H-NMR(CDCl₃); 3.46(s,6H), 3.53(t,2H), 4.50(t,1H), 6.21(s,1H), 6.80-7.10(m,1H), 7.20-7.70(m,12H)

### Working Example 3

### Synthesis of 2-[4-(3-Acetylbenzyl)-3,5-dichlorophenyl]-1-benzylidene-4-(2,2-dimethoxyethyl)semicarbazide

In 8 ml of acetonitrile was dissolved 0.8 g of 2-[4-(4-Acetylbenzyl)-3,5-dichlorophenyl]-1-benzylidenehydrazine. To the solution were added 0.4 g 2,2-dimethoxyethylisocyanate and 0.015 g of DBU. The mixture was stirred for one hour at room temperature. The reaction mixture was poured into 30 ml ice-water followed by extraction with ethylacetate. The extract was washed with water, dried over anhydrous magnesium sulfate and concentrated. This residue was purified by column chromatography (Merck silica 60; normal hexane-ethylacetate=1:3) to provide 0.73 g of the title compound as a light-yellow oil.
¹H-NMR(CDCl₃); 2.58(s,3H), 3.47(s,6H), 3.53(5,2H), 4.15(5,1H), 4.43(s,2H), 6.90-7.90(m,13H)

### Working Example 4

Compounds produced by substantially the same manner as in Working Example 1 - 3 and their physical constants were set forth in Table 1.

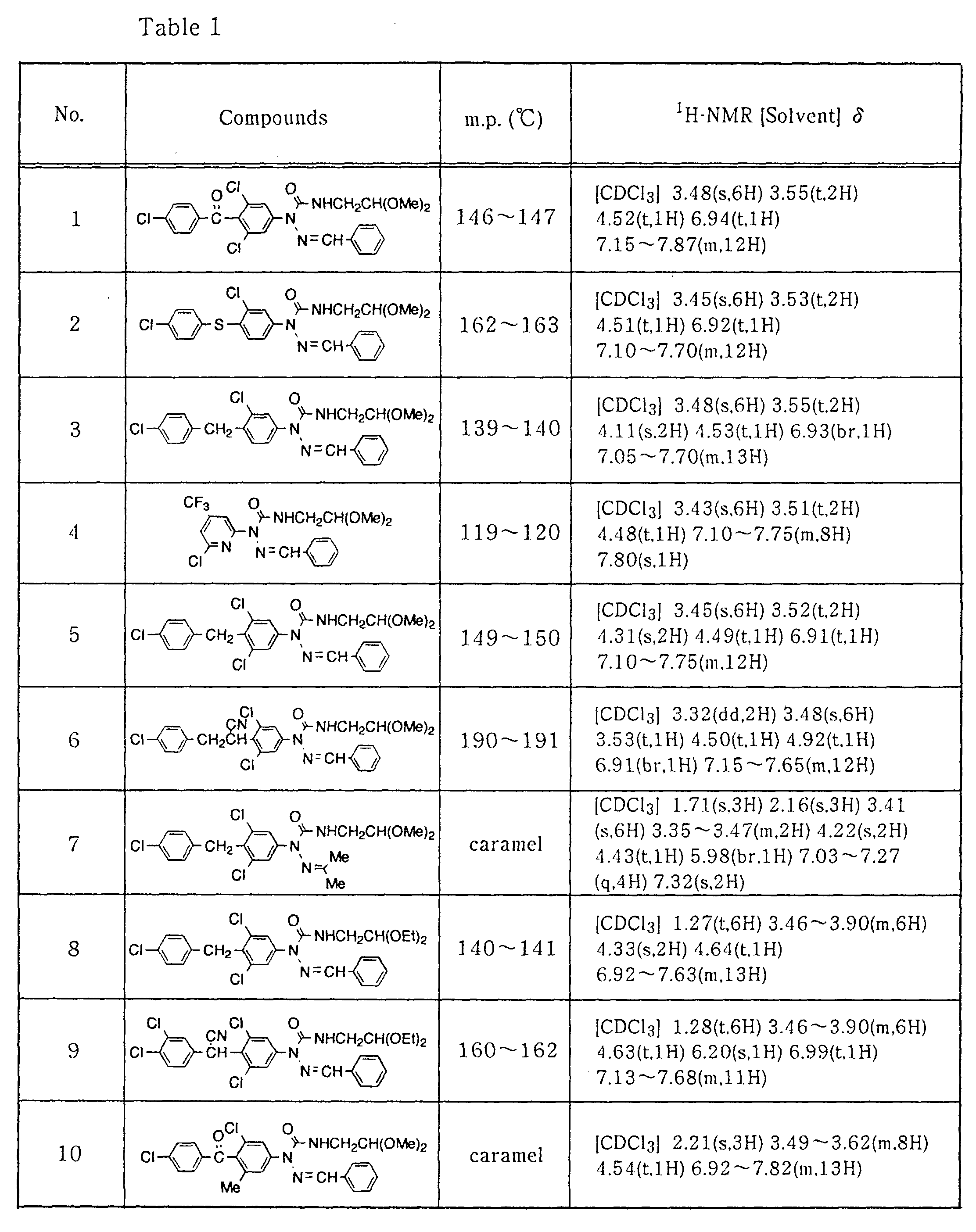

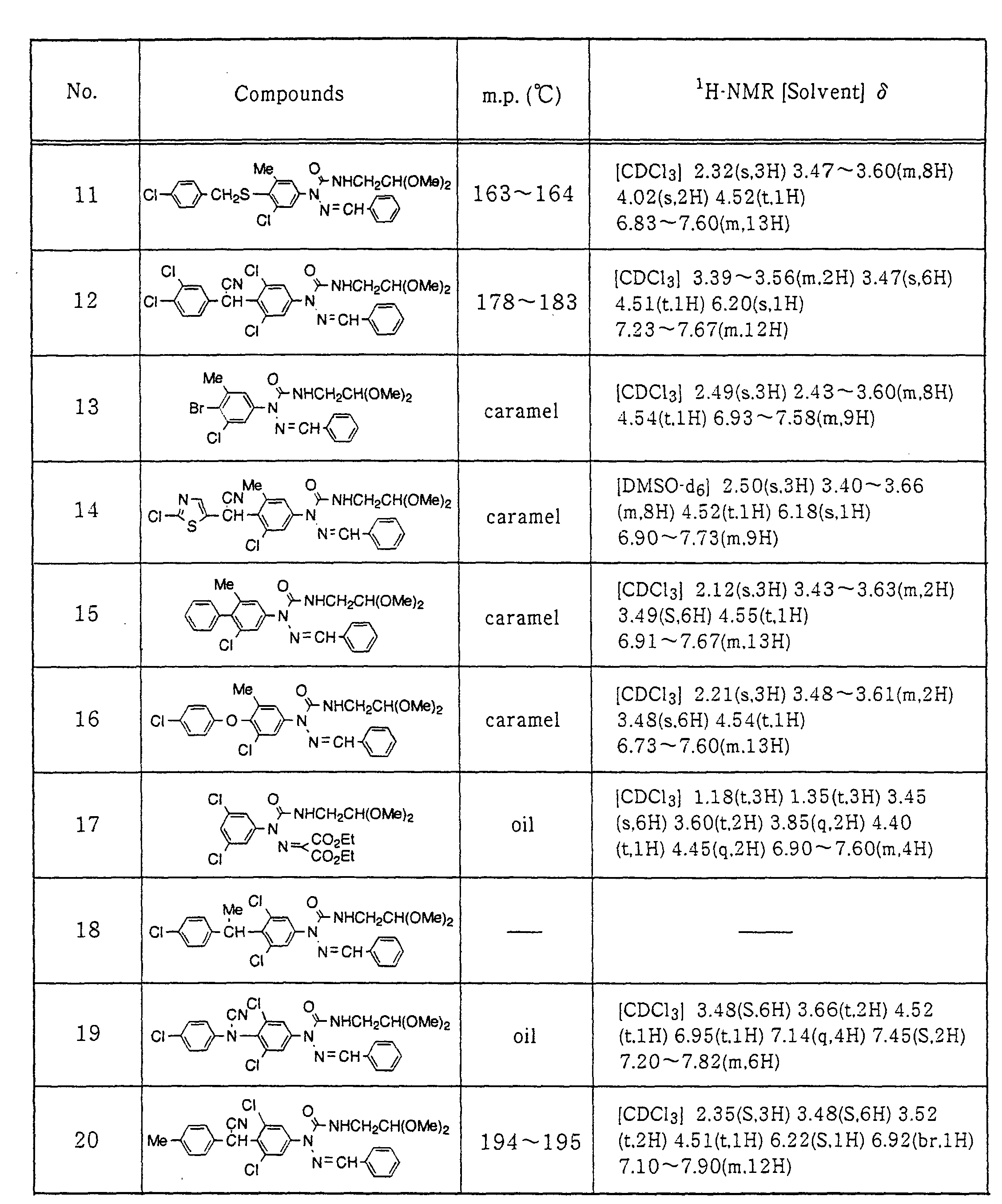

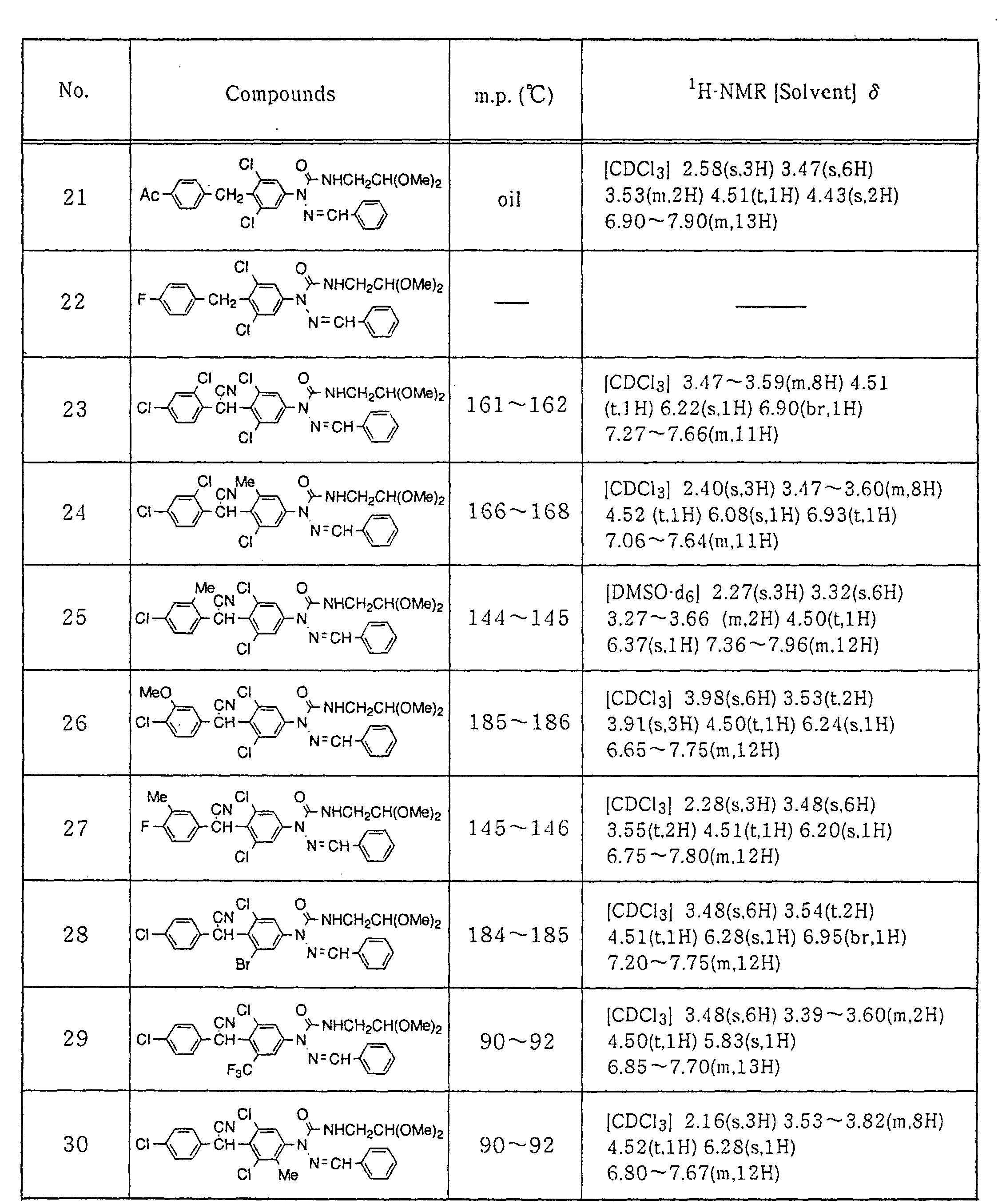

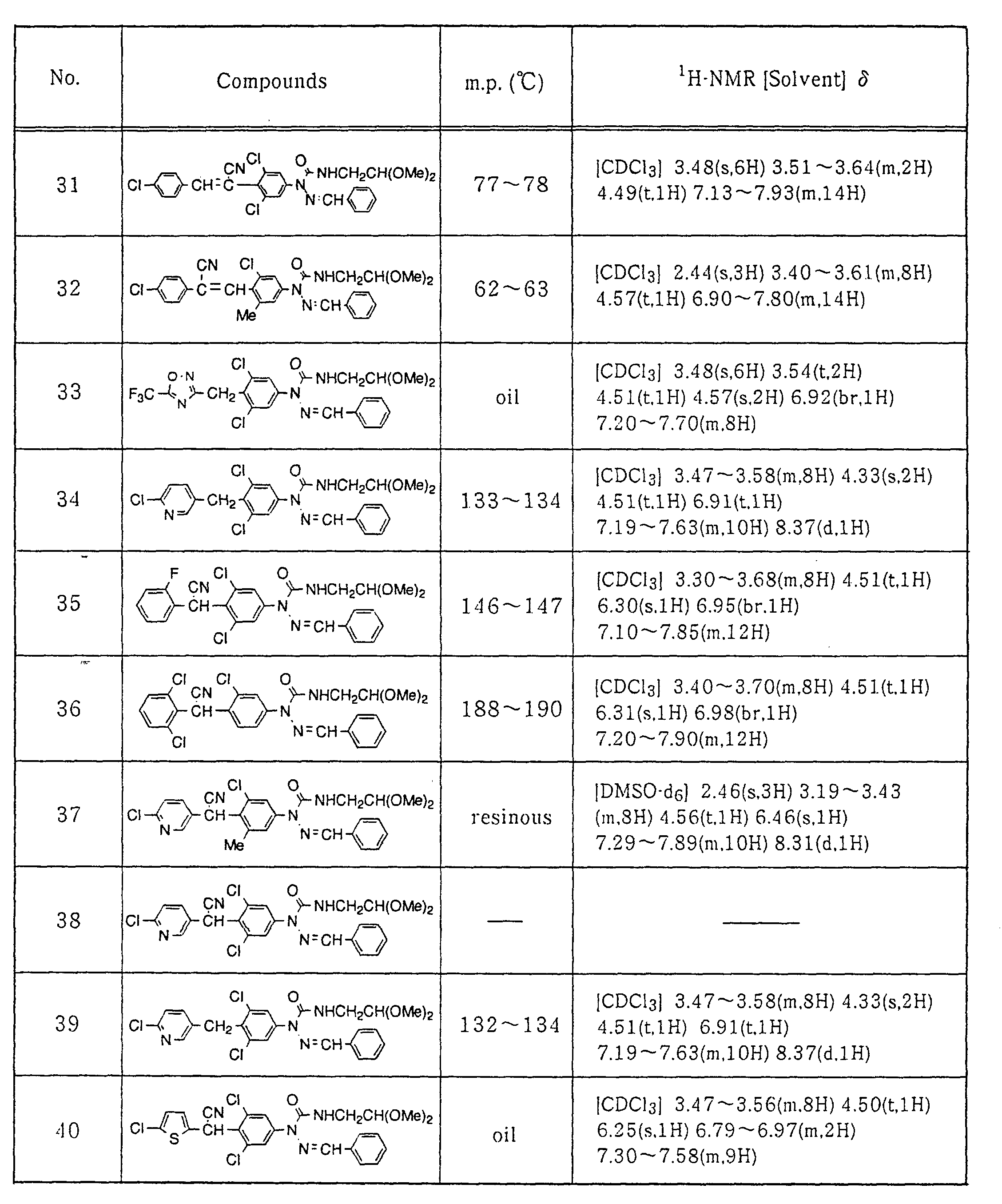

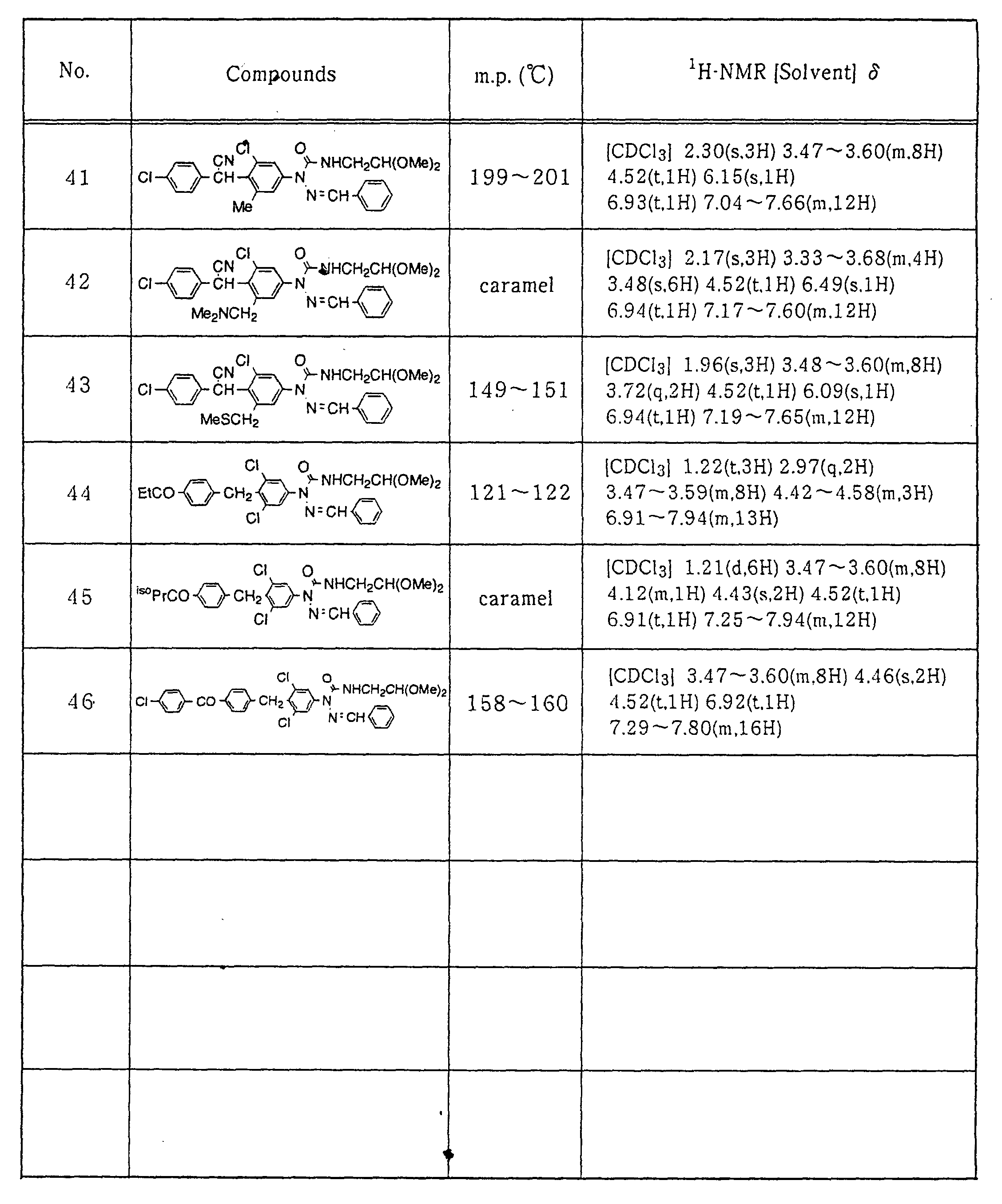

### Working Example 5

### Synthesis of 2-[3,5-dichloro-4-(4-chlorobenzyl)phenyl]-4,5-dihydro-1,2,4-triazin-3(2H)-one

In 5 ml of acetonitrile was dissolved 0.4 g of 1-benzylidene-2-[4-(4-chlorobenzyl)-3,5-dichlorophenyl]-4-(2,2-diethoxyethyl)-semicarbazide. To the solution was added one drop of 35% hydrochloric acid. The mixture was stirred for one hour at room temperature. The reaction mixture was cooled, then resulting crystalline precipitate of the titled compound was collected by filtration. The yield was 95%.
m.p.199-200°C
¹H-NMR(CDCl₃); 4.05(t,2H), 4.25(s,2H), 6.50(br,1H), 7.05(t,1H), 7.19(s,4H), 7.60(s,2H)

### Working Example 6

### Synthesis of 2-[4-(4-acetylbenzyl)-3,5-dichlorophenyl]-4,5-dihydro-1,2,4-triazin-3(2H)-one

In 7 ml of ethyl acetate was dissolved 0.7 g of 2-[4-(4-acetylbenzyl)-3,5-dichlorophenyl]-1-benzylidene-4-(2,2-dimethoxyethyl)semicarbazide. To the solution was added 0.27 g of 35% hydrochloric acid. The mixture was stirred for one hour at room temperature. The reaction mixture was washed with water, dried and concentrated. The residue was purified by column chromatography (Merck Silica Gel 60; normalhexane-ethylacetate=1:3) to provide 0.24 g of the title compound. m.p.189-190°C
¹H-NMR(CDCl₃); 2.56(s.3H), 4.14(5,2H), 4.35(s,2H), 5.60(br,1H), 7.11(m,1H), 7.28(d,2H), 7.62(s,2H), 7.85(d,2H)

### Working Example 7

Compounds produced by substantially the same manner as in Working Example 5 and their physical constants were set forth in Table 2.

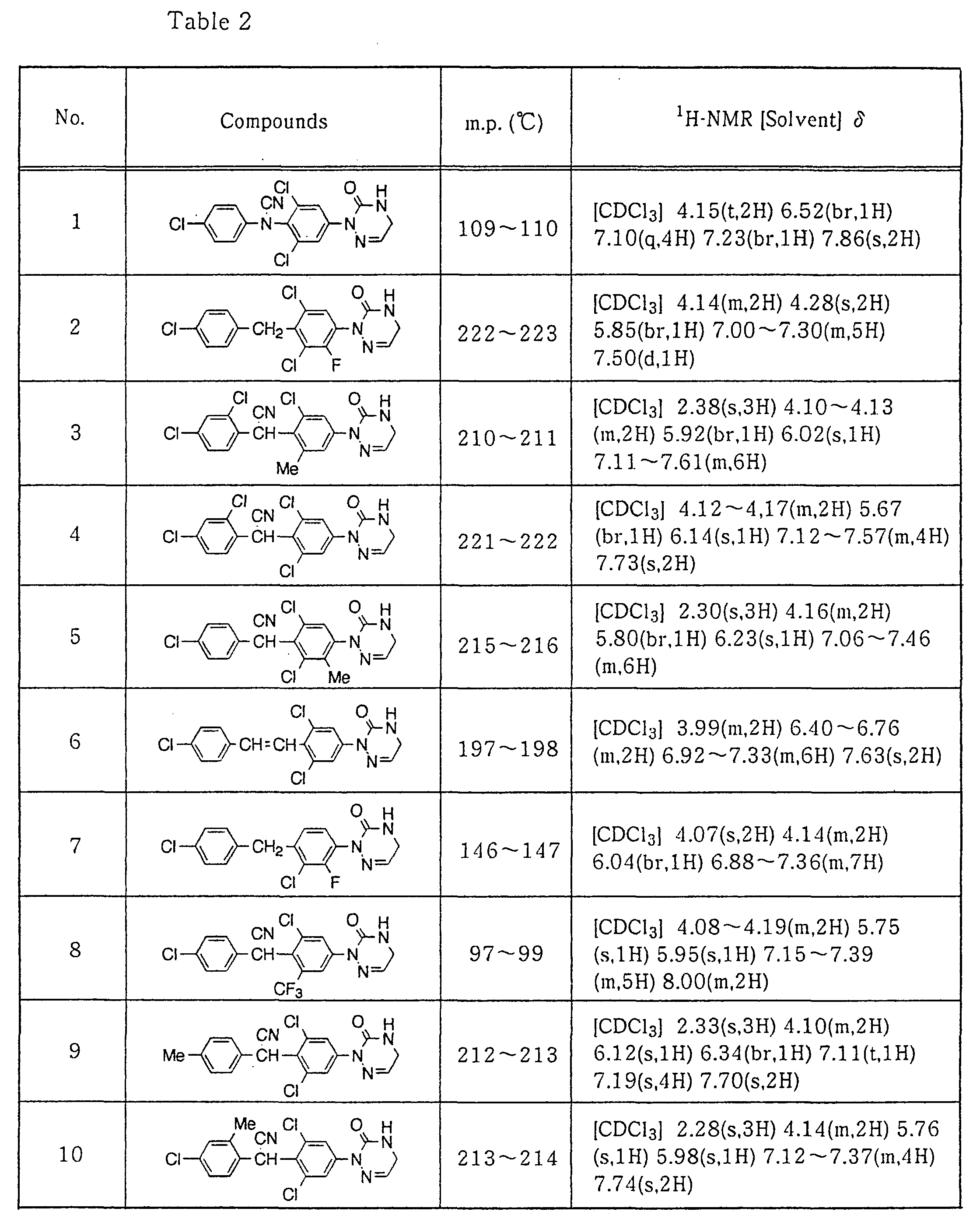

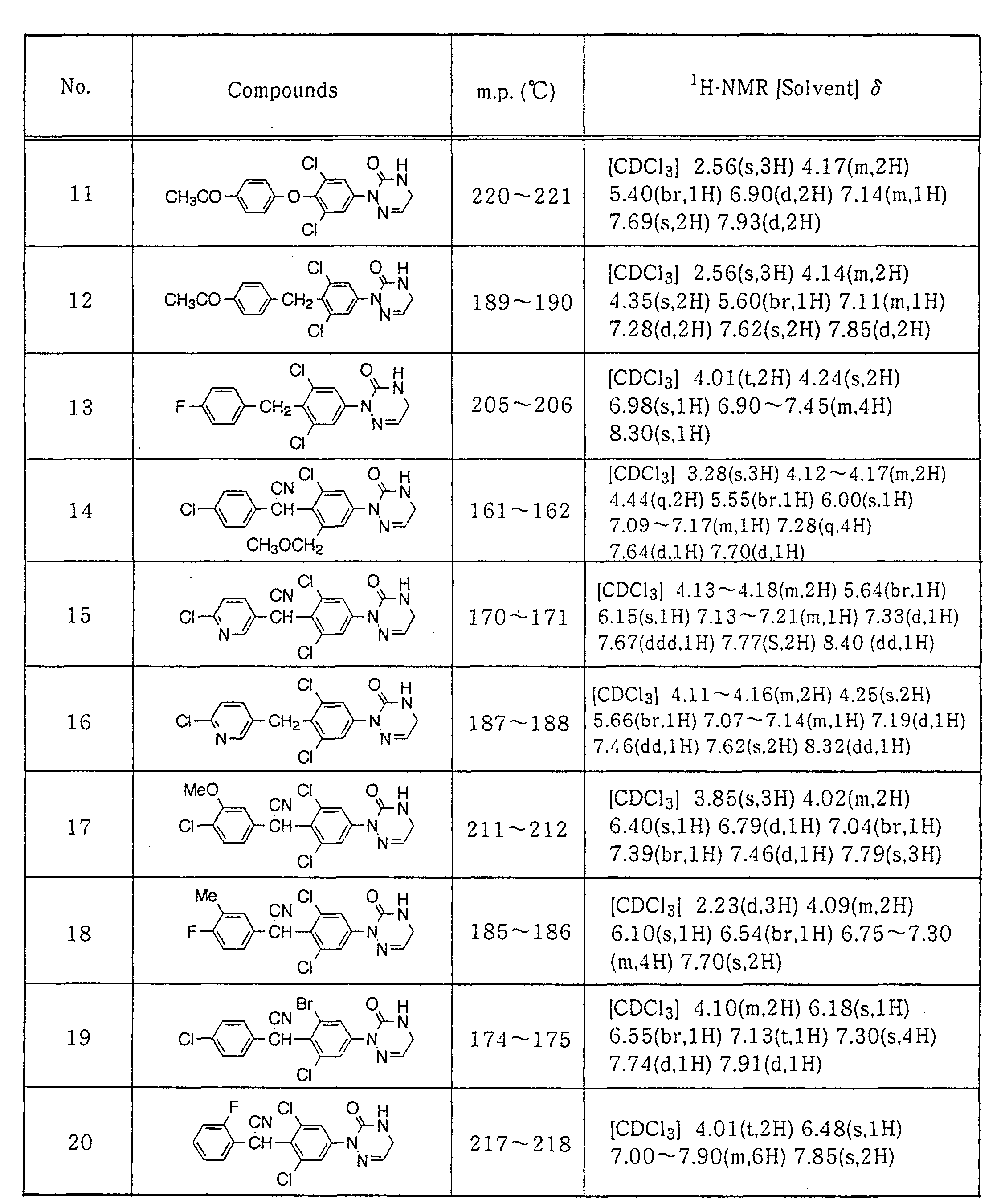

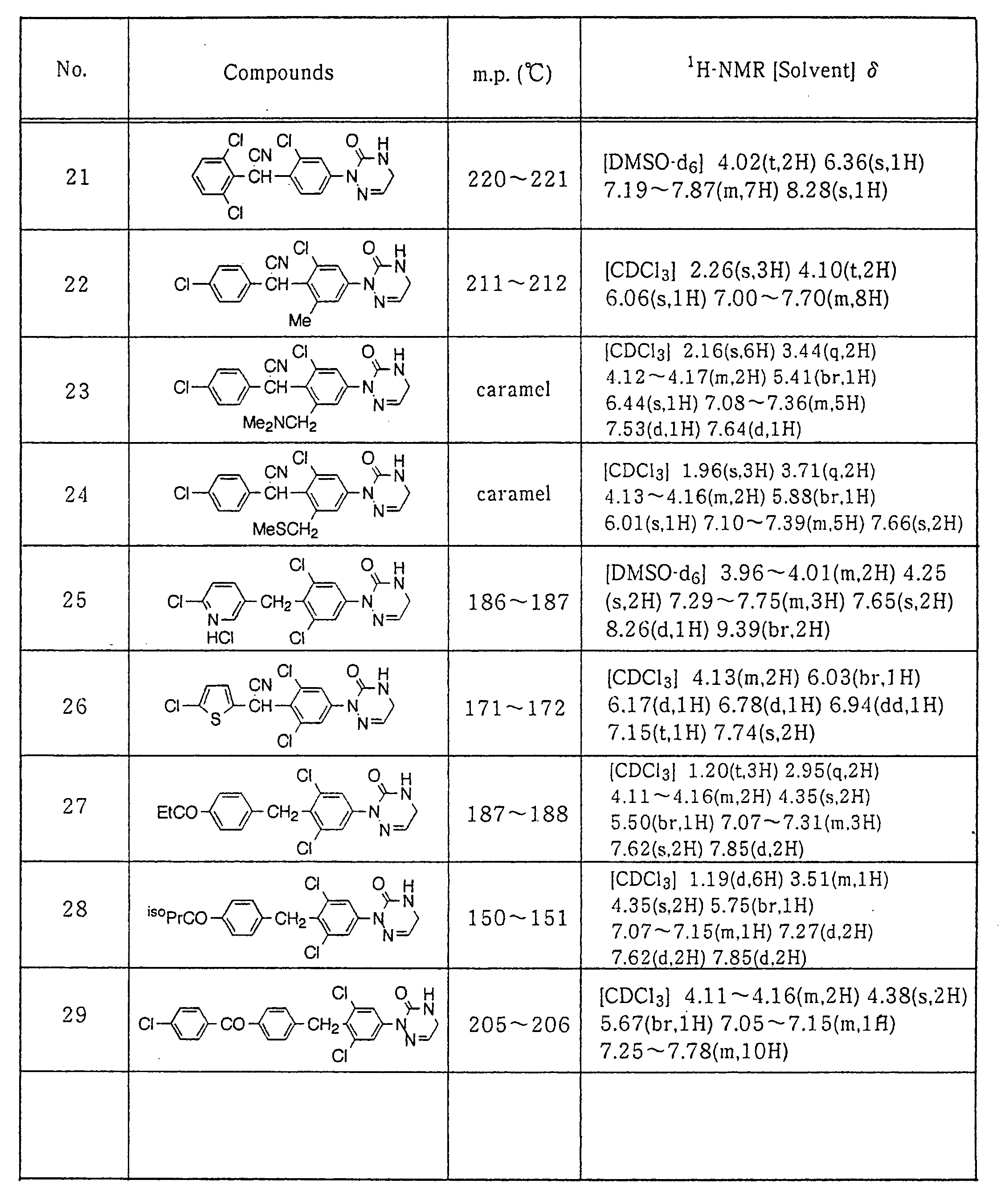

### Working Example 8

### Synthesis of 2-[3,5-dichloro-4-(4-chlorobenzyl)phenyl]-1,2,4-triazine-3,5(2H,4H)-dione

In 2 ml of acetic acid were dissolved 0.2 g of 2-[3,5-dichloro-4-(4-chlorobenzyl)phenyl]-4,5-dihydro-1,2,4-triazine-3(2H)-one and 0.2 ml of hydrogenperoxide (30%). The reaction was allowed to proceed for 3 hours at temperatures ranging from 100 to 110°C. To the reaction mixture was added 20 ml of water to cause precipitation of the titled compound as crystalline product. The product was collected by filtration. The yield was 85%.
m.p.175-176°C
¹H-NMR(DMSO-d₆); 4.30(s,2H), 7.25(q,4H), 7.70(s,1H), 7.74(s,2H), 12.46(br,1H)

### Working Example 9

Compounds produced by substantially the same manner as in Working Example 8 and their physical constants were set forth in Table 3.

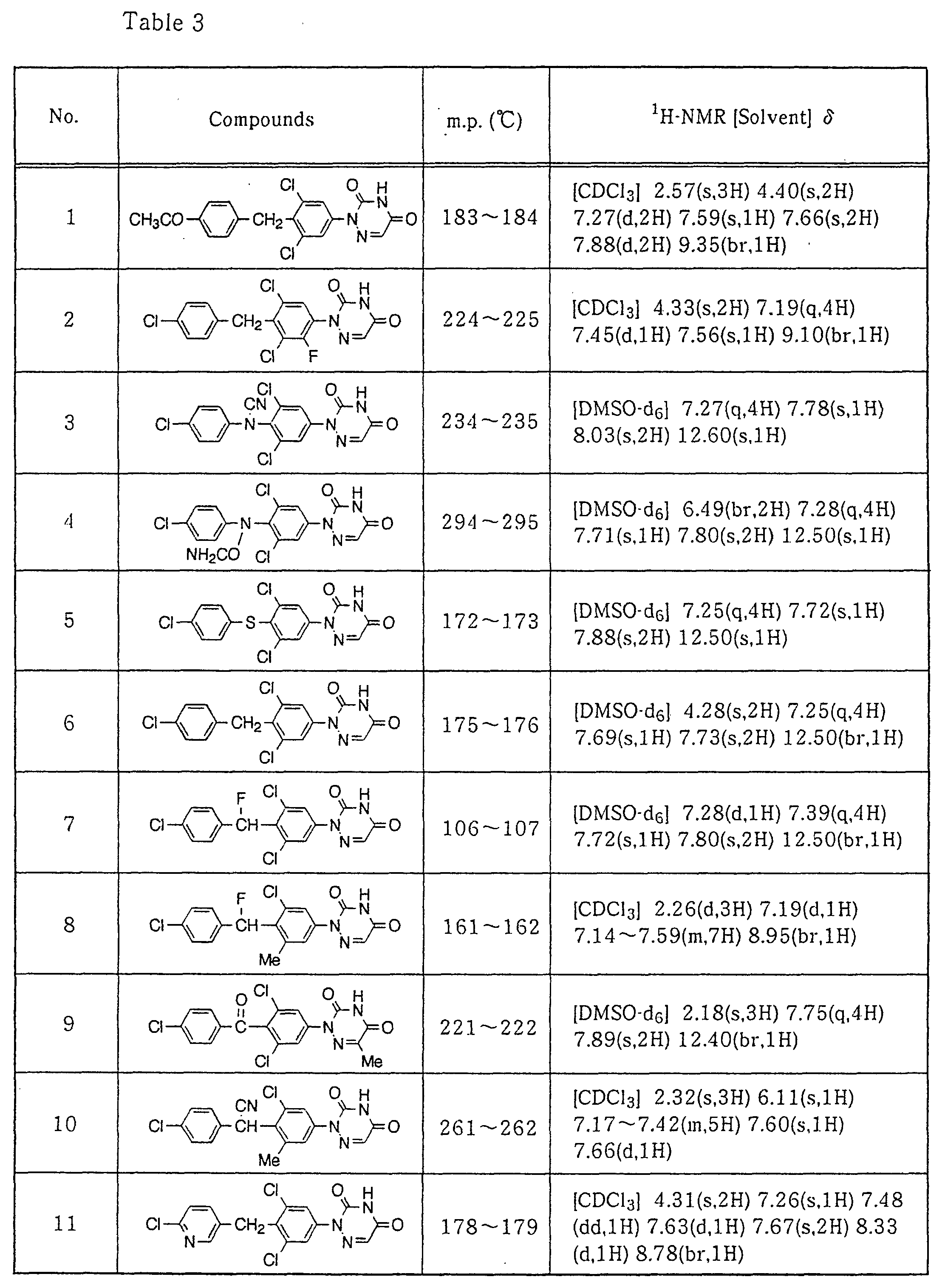

### Working Example 10

### Production of 2-[4-(4-chloro-α-cyanobenzyl)-3,5-dichlorophenyl]-4,5-dihydro-1,2,4-triazin-3(2H)-one

In 30 ml of acetonitrile was suspended 3.5 g of 1-benzylidene-2-[4-(4-chloro-α-cyanobenzyl)-3,5-dichlorophenyl]-4-(2,2-dimethoxyethyl)semicarbazide produced in Working Example 2. To the suspension was added 0.7 g of 35% hydrochloric acid, and the reaction was allowed to proceed for one hour at room temperature. After completion of the reaction, the reaction mixture was cooled for 30 minutes at temperatures ranging from 0 to 10°C, then resulting crystalline precipitate was collected by filtration to afford 2.3 g of the titled compound as colorless crystals, m.p.166-167°C.
¹H-NMR(CDCl₃); 4.09(t,2H), 6.11(s,1H), 6.52(br,1H), 7.12(t,1H), 7.30(s,4H), 7.70(s,2H)

### Working Example 11

### Production of 2-[4-(4-chloro-α-cyanobenzyl)-3,5-dichlorophenyl]-1,2,4-triazine-3,5(2H,4H)-dione

In 20 ml of acetic acid was dissolved 2.0 g of 2-[4-(4-chloro-α-cyanobenzyl)-3,5-dichlorophenyl]-4,5-dihydro-1,2,4-triazin-3(2H)-one. To the solution was added three times as much mol. of 30% hydrogen peroxide. The reaction was allowed to proceed for 3 hours at 100°C. After completion of the reaction, the reaction mixture was poured into ice-water. Resulting crystalline precipitate was collected by filtration to afford 1.8 g of the titled compound as colorless crystals, m.p.290-292°C.
¹H-NMR(DMSO-d₆); 6.53(s,1H), 7.40(q,4H), 7.72(s,1H), 7.85(s,2H), 12.50(s,1H)

### Working Example 12

### Synthesis of 2-[4-(4-chlorobenzyl)-3,5-dichlorophenyl]-1,2,4-triazine-3(2H)-oxo-5(4H)-thione

In 50 ml of toluene was suspended 1.9 g of 2-[4-(4-chlorobenzyl)-3/5-dichlorophenyl]-1,2,4-triazine-3,5(2H,4H)-dione. To the suspension was added 1.2 g of 2,4-Bis(4-methoxyphenyl)-1,3-dithia-2,4-diphosphetane-2,4-disulfide and refluxed for one hour. After completion of the reaction, insolubles were removed and the solution was concentrated. After the concentrate was added diethylether, 1.8 g of the title compound was filtlated as crystals. m.p.173-175°C.

### Working Example 13

Compounds produced by substantially the same manner as in Working Example 12 and their physical constants were set forth in Table 4.

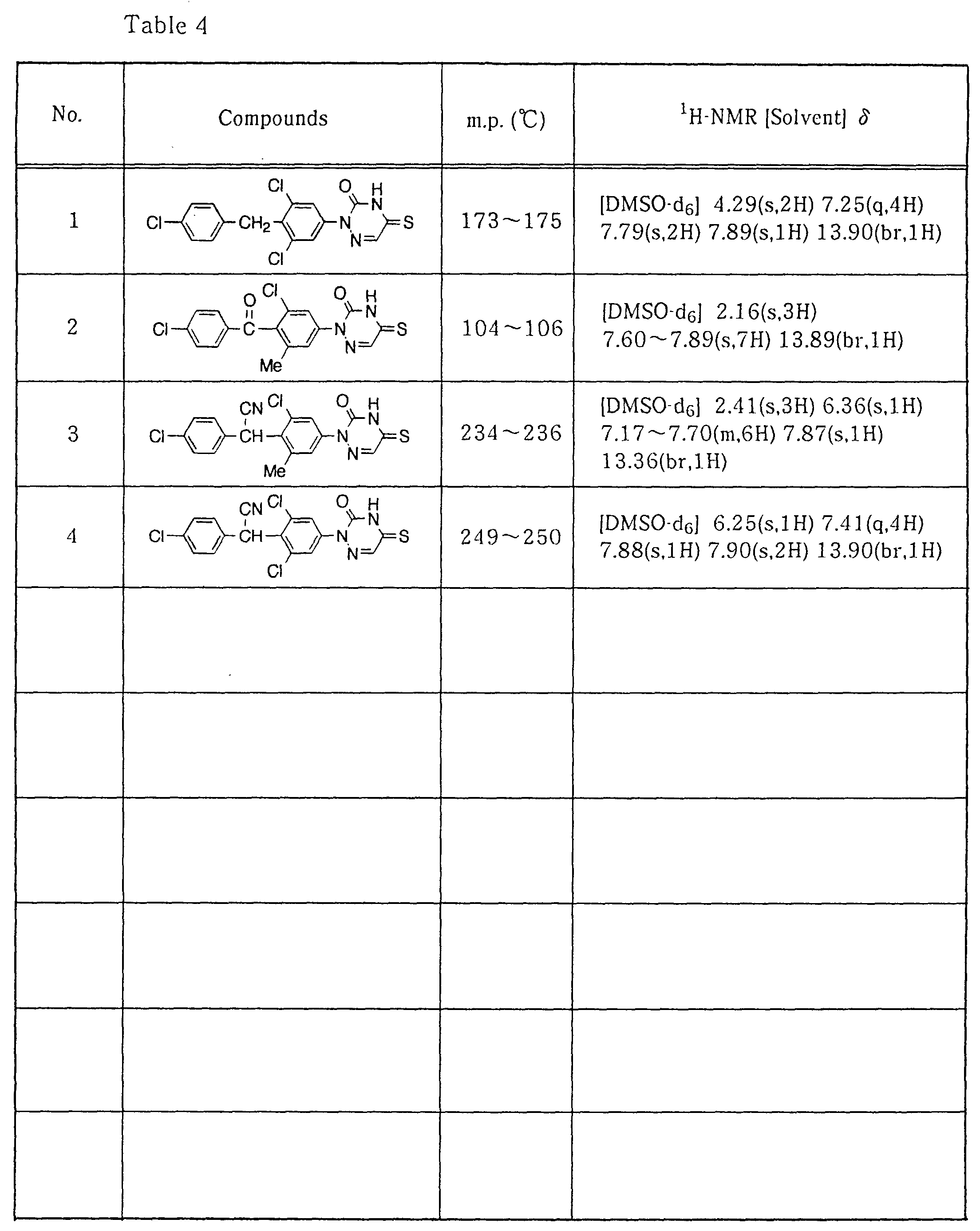

### Working Example 14

### Synthesis of 2-[4-(4-chlorobenzyl)-3,5-dichlorophenyl]-hexahydro-1,2,4-triazine-3,5(3H,4H)-dione.

In 100 ml of acetic acid was dissolved 2.3 g of 2-[4-(4-chlorobenzyl)-3,5-dichlorophenyl]-1,2,4-triazine-3,5(2H,4H)-dione and refluxed with 3.1 g zinc powder for 3 hours. After completion of the reaction, insolubles were removed, and the solution was concentrated. After concentrating was added 20 ml iced water, then the resulting crystalline precipitate was collected by filtration, which was washed with methanol, followed by drying to afford 2.1 g of the title compound as colorless crystals, m.p.267-268°C.

### Working Example 15

Compounds produced by substantially the same manner as in Working Example 14 and their physical constants were set forth in Table 5.

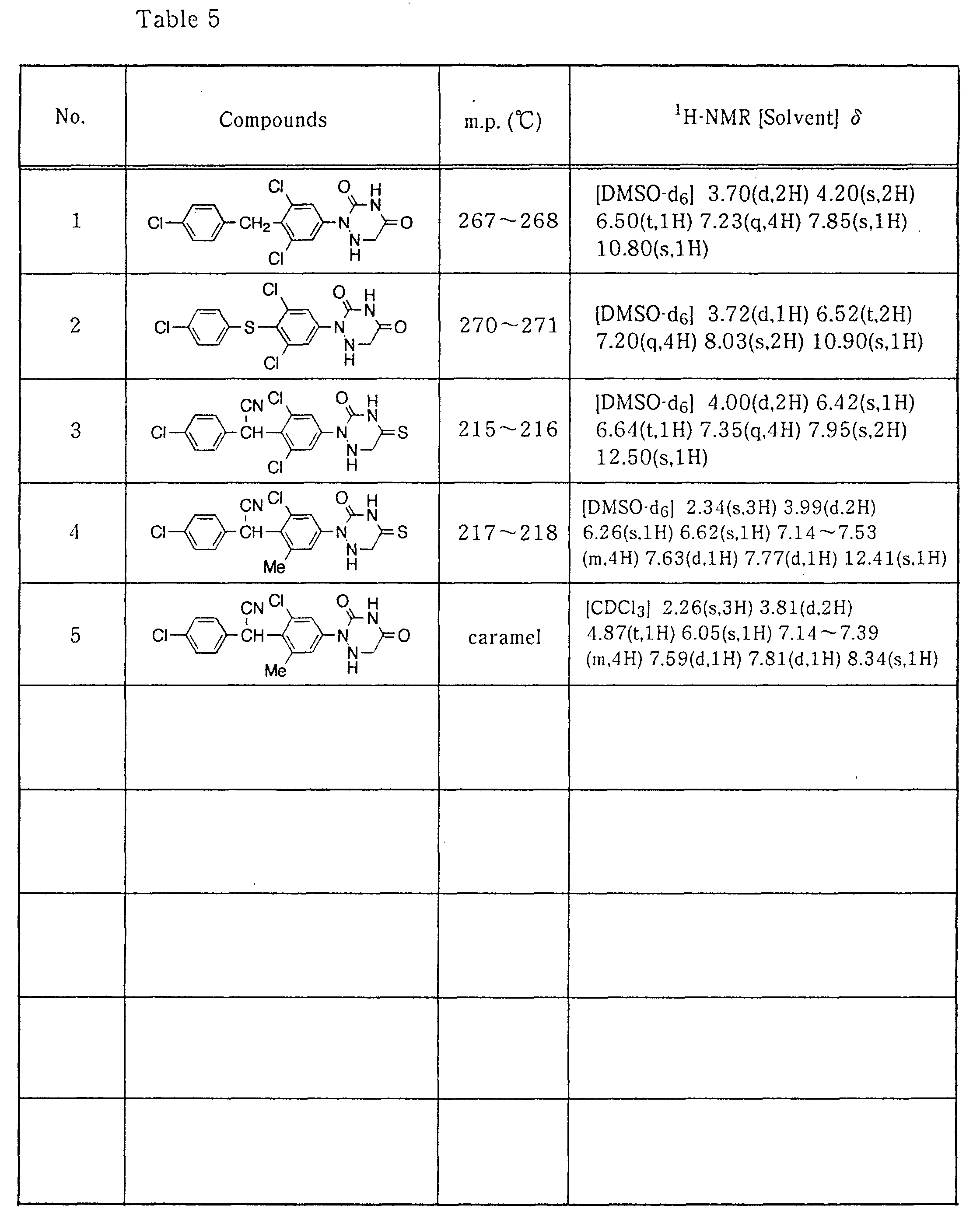

### Working Example 16

### Synthesis of 2-{4-[4-(1-hydroxyethyl)benzyl]-3,5-dichlorophenyl}-4,5-dihydro-1,2,4-triazin-3(2H)-one

In 1.5 ml of ethanol was suspended 0.15 g of 2-[4-(4-acetylbenzyl)-3,5-dichlorophenyl]-4,5-dihydro-1,2,4-triazin-3(2H)-one. To the suspension was added 0.1 g of sodium borohydride. The mixture was stirred for one hour at room temperature. The reaction mixture was poured into 10 ml ice-water, followed by extraction with 10 ml of ethylacetate. The extract was dried over anhydrous magnesium sulfate, and concentrated. This residue was purified by column chromatography (Merck Silica Gel 60; hexane-acetone=1:1) to provide 0.13 g of the title compound as colorless crystals,
m.p.119-120°C.
¹H-NMR(CDCl₃); 1.52(d,3H), 2.58(br,1H), 4.12(m,2H), 4.29(s,2H), 4.81-4.96(m,1H), 5.29(br,1H), 7.05-7.33(m,5H), 7.59(s,2H)

### Working Example 17

### Synthesis of 2-{4-[4-(1-hydroxypropyl)benzyl]-3,5-dichlorophenyl}-4,5-dihydro-1,2,4-triazine-3(2H)-one

The title compound was synthesized in otherwise a similar manner as Example 16, m.p.148-149°C.
¹H-NMR(CDCl₃) ; 0.89(t,3H),. 1.57-1.84(m,2H), 2.56(br,1H), 4.11(m,2H), 4.29(s,2H), 4.54(m,1H), 5.56(br,1H), 7.05-7.20(n,5H), 7.59(s,2H)

### Working Example 18

### Synthesis of 2-[4-(4-chlorobenzyl)-3,5-dichlorophenyl]-1,2,4-triazine-3,5(2H,4H)-dione.

In 30 ml of tetrahydrofuran was dissolved 2.6 g of 2-[4-(4-chlorobenzyl)-3,5-dichlorophenyl]-4,5-dihydro-1,2,4-triazin-3(2H)-one. To the solution was added 4.5 g of pyridinium chlorochromate. The mixture was stirred at room temperature overnight, after which the insoluble matter was filtered off. The filtrate was concentrated. The residue was purified by column chromatography (Merck Silica Gel 60; chloroformethanol=20:1) to provide 2.2 g of the title compound as colorless crystals, m.p.175-176°C.

The production method of this invention is to produce various 2-substituted-1,2,4-triazin-3-one derivatives, which are useful as, for example, herbicides and agents of controlling parasitic pest, conveniently and in a high yield. These useful compounds have come to be produced on an industrial scale, which makes a great contribution to the introduction of triazine derivatives, which are useful as, for example, medicine, veterinary drugs and agricultural chemicals, into market less expensively. Besides, the present invention contributes a great deal to the creation and development of useful and novel 2-substituted-1,2,4-triazin-3-one derivatives.

## Claims

1. A method of producing a 1,2,4-triazine-3-one derivative represented by the chemical formula: 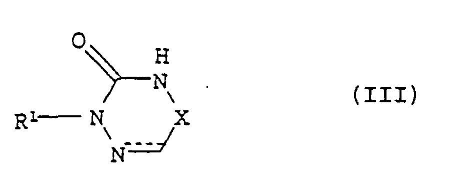 wherein R¹ stands for a C₆₋₁₄ aryl group or a 5- to 6-membered aromatic heterocyclic group, wherein each group may optionally have one to five substituents selected from the group consisting of (1) C₁₋₄ alkyl groups, (2) C₂₋₄ alkenyl groups, (3) C₇₋₁₁ aralkyl groups, (4) phenyl group, (5) C₁₋₆ alkoxy groups, (6) phenoxy group, (7) C₁₋₆ alkanoyl groups, (8) benzoyl group, (9) carboxyl group, (10) C₂₋₇ alkoxycarbonyl groups, (11) carbamoyl group, (12) N-mono-C₁₋₄ alkylcarbamoyl groups, (13) N,N-di-C₁₋₄ alkylcarbamoyl groups, (14) halogen atoms, (15) mono-, di- or tri-halogeno-C₁₋₄ alkyl groups, (16) optionally protected amino groups, (17) mono-C₁₋₄ alkylamino groups, (18) C₁₋₆ alkanoylamino groups, (19) benzoylamino groups, (20) carbamoylamino group, (21) N-C₁₋₄ alkylcarbamoylamino groups, (22) N,N-di-C₁₋₄ alkylcarbamoylamino groups, (23) C₁₋₃ alkylenedioxy groups, (24) hydroxy group, (25) nitro group, (26) cyano group, (27) mercapto group, (28) sulfo group, (29) sulfino group, (30) phosphono group, (31) sulfamoyl group, (32) C₁₋₆ monoalkylsulfamoyl groups, (33) di-C₁₋₄ alkylsulfamoyl groups, (34) C₁₋₆ alkylthio groups, (35) phenylthio group, (36) C₁₋₆ alkylsulfinyl groups, (37) phenylsulfinyl group, (38) C₁₋₆ alkylsulfonyl groups, (39) phenylsulfonyl group and (40) a 5- or 6-membered heterocyclic group containing 1 to 4 hetero-atoms selected from oxygen atom, sulfur atom and nitrogen atom, which may optionally be linked to the above-mentioned C₆₋₁₄ aryl group or 5- to 6-membered aromatic heterocyclic group through an atomic chain consisting of 1 to 4 carbon atoms, oxygen atoms and nitrogen atoms, further above-mentioned groups having carbon chain or cyclic group containing two or more carbon atoms may optionally have one to four substituents selected from the group consisting of (a) halogen atoms, (b) hydroxy group, (c) C₁₋₄ alkoxy groups or oxo group, (d) di-C₁₋₄ alkylamino groups, (e) halogeno-C₁₋₄ alkyl groups, (f) C₁₋₇ acyl groups, (g) hydroxy-C₁₋₄ alkyl groups, (h) C₁₋₄ alkoxy-C₁₋₄ alkyl groups, (i) sulfamoyl and C₁₋₅ sulfamoyl groups, (j) carbamoyl and C₁₋₇ carbamoyl group, (k) C₂₋₄ alkyl groups, (1) carboxyl group, (m) C₁₋₇ alkoxycarbonyl group and phenoxycarbonyl;
X stands for carbonyl group, thiocarbonyl group or methylene group; and dashed line means that a double bond may optionally be formed; which comprises
(i) allowing a hydrazone derivative represented by the chemical formula: 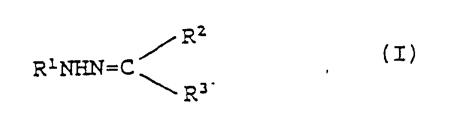 wherein R¹ has the same meaning as defined above; and R² and R³ stand for (1) hydrogen, (2) hydrocarbon residual group selected from the group consisting of C₁₋₄ alkyl groups, aromatic homocyclic groups and 5- or 6-membered aromatic heterocyclic groups, or (3) an electron withdrawing group selected from the group consisting of cyano group, C₁₋₆ alkoxy-carbonyl, hydroxycarbonyl, C₆₋₁₀ aryloxy-carbonyl groups, a 5- or 6-membered heterocyclic-oxycarbonyl group containing 1 to 4 hetero-atoms selected from nitrogen atom, sulfur atom and oxygen atom, C₁₋₆ alkylsulfonyl groups which may optionally be substituted with one to three halogen atoms, aminosulfonyl, di-C₁₋₄ alkoxyphosphoryl groups, C₁₋₆ acyl groups which may optionally be substituted with one to three halogen atoms, carbamoyl and C₁₋₆ alkylsulfonylthiocarbamoyl groups, to react with dialkoxyethyl isocyanate represented by (R⁴O)₂CHCH₂NCO wherein R⁴ stands for a C₁₋₄ alkyl group or one R⁴ and the other R⁴ may be optionally combine with each other to form a ring with an alkylene chain, to give the semicarbazone derivative represented by the chemical formula: 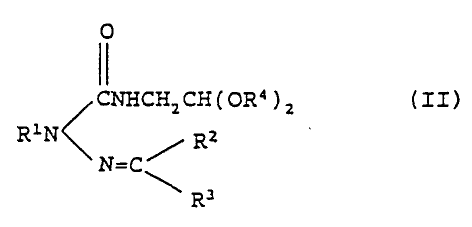 wherein respective groups have the same meanings as defined above, and then
(ii)subjecting the semicarbazone derivative to a ring-closure reaction.

2. The method of producing a 1,2,4-triazine-3-one derivative as claimed in claim 1, wherein R¹ is phenyl group which may optionally have one to five substituents selected from the group consisting of (1) C₁₋₄ alkyl groups, (2) C₂₋₄ alkenyl groups, (3) C₇₋₁₁ aralkyl groups, (4) phenyl group, (5) C₁₋₆ alkoxy groups, (6) phenoxy group, (7) C₁₋₆ alkanoyl groups, (8) benzoyl group, (9) carboxyl group, (10) C₂₋₇ alkoxycarbonyl groups, (11) carbamoyl group, (12) N-mono-C₁₋₄ alkylcarbamoyl groups, (13) N,N-di-C₁₋₄ alkylcarbamoyl groups, (14) halogen atoms, (15) mono-, di- or tri-halogeno-C₁₋₄ alkyl groups, (16) optionally protected amino groups, (17) mono-C₁₋₄ alkylamino groups, (18) C₁₋₆ alkanoylamino groups, (19) benzoylamino groups, (20) carbamoylamino group, (21) N-C₁₋₄ alkylcarbamoylamino groups, (22) N,N-di-C₁₋₄ alkylcarbamoylamino groups, (23) C₁₋₃ alkylenedioxy groups, (24) hydroxy group, (25) nitro group, (26) cyano group, (27) mercapto group, (28) sulfo group, (29) sulfino group, (30) phosphono group, (31) sulfamoyl group, (32) C₁₋₆ monoalkylsulfamoyl groups, (33) di-C₁₋₄ alkylsulfamoyl groups, (34) C₁₋₆ alkylthio groups, (35) phenylthio group, (36) C₁₋₆ alkylsulfinyl groups, (37) phenylsulfinyl group, (38) C₁₋₆ alkylsulfonyl groups, (39) phenylsulfonyl group and (40) a 5- or 6-membered heterocyclic group containing 1 to 4 hetero-atoms selected from oxygen atom, sulfur atom and nitrogen atom, which may optionally be linked to the above-mentioned phenyl group through an atomic chain consisting of 1 to 4 carbon atoms, oxygen atoms and nitrogen atoms, further above-mentioned groups having carbon chain or cyclic group containing two or more carbon atoms may optionally have one to four substituents selected from the group consisting of (a) halogen atoms, (b) hydroxy group, (c) C₁₋₄ alkoxy groups or oxo group, (d) di-C₁₋₄ alkylamino groups, (e) halogeno-C₁₋₄ alkyl groups, (f) C₁₋₇ acyl groups, (g) hydroxy-C₁₋₄ alkyl groups, (h) C₁₋₄ alkoxy-C₁₋₄ alkyl groups, (i) sulfamoyl and C₁₋₅ sulfamoyl groups, (j) carbamoyl and C₁₋₇ carbamoyl group, (k) C₂₋₄ alkyl groups, (1) carboxyl group, (m) C₁₋₇ alkoxycarbonyl group and phenoxycarbonyl.

3. The method of producing a 1,2,4-triazine-3-one derivative as claimed in Claim 2, wherein the phenyl group is a phenyl group substituted at the positions 3 and 4, or a phenyl group substituted at the positions 3, 4 and 5.

4. The method of producing a 1,2,4-triazine-3-one derivative as claimed in Claim 1, wherein R⁴ is C₁₋₄ alkyl.

5. The method of producing a 1,2,4-triazine-3-one derivative as claimed in Claim 4, wherein R⁴ is methyl or ethyl.

6. The method as claimed in Claim 1, which comprises production of a 1,2,4-triazine-3,5-dione derivative.

7. The method as claimed in Claim 1, which comprises production of a hexahydro-1,2,4-triazine-3-one derivative.

8. The method as claimed in Claim 1, which comprises production of 2-[4-(4-acetylbenzyl)-3,5-dichlorophenyl]-4,5-dihydro-1,2,4-triazine-3(2H)-one.

9. The method as claimed in Claim 1, which comprises production of 2-{3,5-dichloro-4-[4-(1-hydroxyethyl)-benzyl]phenyl}-4,5-dihydro-1,2,4-triazine-3(2H)-one.

10. The method as claimed in Claim 1, wherein R¹ is a group of the chemical formula: 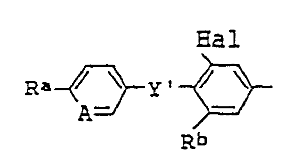 wherein R^{a} is a C₁₋₇ acyl, a sulfamoyl and C₁₋₅ sulfamoyl groups, aminocarbonyl, carbamoyl and C₁₋₇ carbamoyl-groups, a C₁₋₇ alkoxycarbonyl, a C₂₋₄ alkyl, halogeno-C₁₋₄ alkyl, hydroxy-C₁₋₄ alkyl, C₁₋₄ alkoxy-C₁₋₄ alkyl, amino, or di-C₁₋₄ alkylamino; A is -CH- or nitrogen atom; Y' is methylene, cyanomethylene, carbonyl, hydroxymethylene, sulfur atom, sulfinyl, sulfonyl or oxygen atom; Hal is halogen; and R^{b} is hydrogen, halogen or lower alkyl group.

11. A 1,2,4-triazine-3-one derivative represented by the chemical formula: 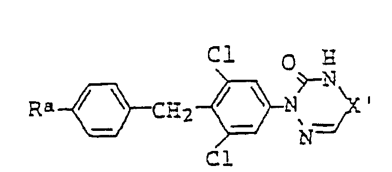 wherein R^{a} is a C₁₋₇ acyl, a sulfamoyl and C₁₋₅ sulfamoyl, a C₂₋₄ alkyl, halogeno-C₁₋₄ alkyl, hydroxy-C₁₋₄ alkyl, or C₁₋₄ alkoxy-C₁₋₄ alkyl, and X' is methylene or carbonyl.

12. The 1,2,4-triazine-3-one derivative as claimed in claim 11, which is 2-[4-(4-Acetylbenzyl)-3,5-dichlorophenyl]-4,5-dihydro-1,2,4-triazine-3(2H) -one.

13. The 1,2,4-triazine-3-one derivative as claimed in claim 11, which is 2-(3,5Dichloro-4-[4-(1-hydroxyethyl)-benzyl]phenyl)-4,5-dihydro-1,2,4-triazine-3(2H)-one.

## Patentansprüche

1. Verfahren zur Herstellung eines 1,2,4-Triazin-3-on-Derivats, das durch die chemische Formel: 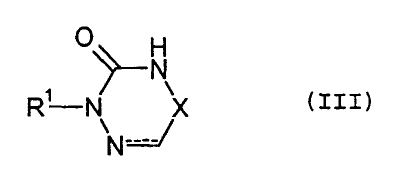 dargestellt wird, wobei
R¹ für eine C₆₋₁₄-Arylgruppe oder eine 5- bis 6-gliedrige aromatische heterocyclische Gruppe steht, wobei jede Gruppe optional einen bis fünf Substituenten besitzen kann, ausgewählt aus der Gruppe, bestehend aus (1) C₁₋₄-Alkylgruppen, (2) C₂₋₄-Alkenylgruppen, (3) C₇₋₁₁-Aralkylgruppen, (4) Phenylgruppe, (5) C₁₋₆-Alkoxygruppen, (6) Phenoxygruppe, (7) C₁₋₆-Alkanoylgruppen, (8) Benzoylgruppe, (9) Carboxylgruppe, (10) C₂₋₇-Alkoxycarbonylgruppen, (11) Carbamoylgruppe, (12) N-Mono-C₁₋₄-alkylcarbamoylgruppen, (13) N,N-Di-C₁₋₄-alkylcarbamoylgruppen, (14) Halogenatomen, (15) Mono-, Di- oder Trihalogen-C₁₋₄-alkylgruppen, (16) optional geschützten Aminogruppen, (17) Mono-C₁₋₄-alkylaminogruppen, (18) C₁₋₆-Alkanoylaminogruppen, (19) Benzoylaminogruppen, (20) Carbamoylaminogruppe, (21) N-C₁₋₄-Alkylcarbamoylaminogruppen, (22) N,N-Di-C₁₋₄-alkylcarbamoylaminogruppen, (23) C₁₋₃-Alkylendioxygruppen, (24) Hydroxygruppe, (25) Nitrogruppe, (26) Cyanogruppe, (27) Mercaptogruppe, (28) Sulfogruppe, (29) Sulfinogruppe, (30) Phosphonogruppe, (31) Sulfamoylgruppe, (32) C₁₋₆-Monoalkylsulfamoylgruppen, (33) Di-C₁₋₄-alkylsulfamoylgruppen, (34) C₁₋₆-Alkylthiogruppen, (35) Phenylthiogruppe, (36) C₁₋₆-Alkylsulfinylgruppen, (37) Phenylsulfinylgruppe, (38) C₁₋₆-Alkylsulfonylgruppen, (39) Phenylsulfonylgruppe und (40) einer 5- oder 6-gliedrigen heterocyclischen Gruppe, welche 1 bis 4 Heteroatome enthält, die ausgewählt sind aus Sauerstoffatom, Schwefelatom und Stickstoffatom, welche optional über eine Atomkette, die aus 1 bis 4 Kohlenstoffatomen, Sauerstoffatomen und Stickstoffatomen besteht, mit der oben erwähnten C₆₋₁₄-Arylgruppe oder 5- bis 6-gliedrigen aromatischen heterocyclischen Gruppe verbunden sein können, wobei ferner die oben erwähnten Gruppen mit einer Kohlenstoffkette oder einer cyclischen Gruppe, welche zwei oder mehrere Kohlenstoffatome enthalten, optional einen bis vier Substituenten besitzen können, der aus der Gruppe ausgewählt ist, bestehend aus (a) Halogenatomen, (b) Hydroxygruppe, (c) C₁₋₄-Alkoxygruppen oder Oxogruppe, (d) Di-C₁₋₄-alkylaminogruppen, (e) Halogen-C₁₋₄-Alkylgruppen, (f) C₁₋₇-Acylgruppen, (g) Hydroxy-C₁₋₄-alkylgruppen, (h) C₁₋₄-Alkoxy-C₁₋₄-alkylgruppen, (i) Sulfamoyl- und C₁₋₅-Sulfamoylgruppen, (j) Carbamoyl- und C₁₋₇-Carbamoylgruppe, (k) C₂₋₄-Alkylgruppen, (1) Carboxylgruppe, (m) C₁₋₇-Alkoxycarbonylgruppe und Phenoxycarbonyl;
X für eine Carbonylgruppe, Thiocarbonylgruppe oder Methylengruppe steht; und
eine gestrichelte Linie bedeutet, daß optional eine Doppelbindung ausgebildet sein kann;
welches umfaßt
(i) Gestatten, daß ein Hydrazonderivat, das durch die chemische Formel: 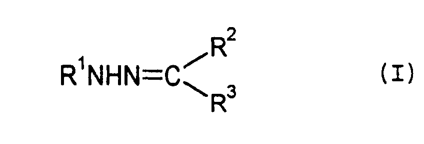 dargestellt ist, wobei
R¹ dieselbe wie oben definierte Bedeutung besitzt und R² und R³ für (1) Wasserstoff, (2) Kohlenwasserstoffrestgruppe, ausgewählt aus der Gruppe, bestehend aus C₁₋₄-Alkylgruppen, aromatischen homocyclischen Gruppen und 5- oder 6-gliedrigen aromatischen heterocyclischen Gruppen, oder (3) eine elektronenabziehende Gruppe, die aus der Gruppe ausgewählt ist, bestehend aus Cyanogruppe, C₁₋₆-Alkoxycarbonyl, Hydroxycarbonyl, C₆₋₁₀-Aryloxycarbonylgruppen, einer 5- oder 6-gliedrigen heterocyclischen Oxycarbonylgruppe, welche 1 bis 4 Heteroatome enthält, die ausgewählt sind aus Stickstoffatom, Schwefelatom und Sauerstoffatom, C₁₋₆-Alkylsulfonylgruppen, die optional mit einem bis drei Halogenatomen substituiert sein können, Aminosulfonyl, Di-C₁₋₄-alkoxyphosphorylgruppen, C₁₋₆-Acylgruppen, die optional mit einem bis drei Halogenatomen substituiert sein können, Carbamoyl und C₁₋₆-Alkylsulfonylthiocarbamoylgruppen, stehen,
mit Dialkoxyethylisocyanat reagiert, welches dargestellt wird durch (R⁴O)₂CHCH₂NCO, wobei R⁴ für eine C₁₋₄-Alkylgruppe steht oder ein R⁴ und das andere R⁴ optional miteinander verbunden sein können, um einen Ring mit einer Alkylenkette zu bilden, um das Semicarbazonderivat zu ergeben, das durch die chemische Formel: 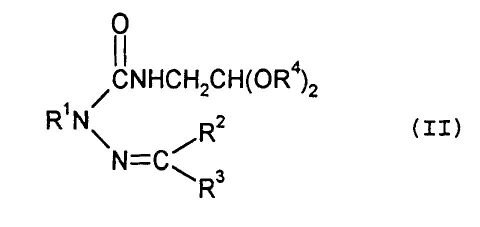 dargestellt wird, wobei entsprechende Gruppen dieselben wie oben definierten Bedeutungen besitzen, und dann
(ii) Unteziehen des Semicarbazonderivats einer Ringschlußreaktion.

2. Verfahren zur Herstellung eines 1,2,4-Triazin-3-on-Derivats nach Anspruch 1, wobei R¹ eine Phenylgruppe ist, die optional einen bis fünf Substituenten besitzen kann, ausgewählt aus der Gruppe, bestehend aus (1) C₁₋₄-Alkylgruppen, (2) C₂₋₄-Alkenylgruppen, (3) C₇₋₁₁-Aralkylgruppen, (4) Phenylgruppe, (5) C₁₋₆-Alkoxygruppen, (6) Phenoxygruppe, (7) C₁₋₆-Alkanoylgruppen, (8) Benzoylgruppe, (9) Carboxylgruppe, (10) C₂₋₇-Alkoxycarbonylgruppen, (11) Carbamoylgruppe, (12) N-Mono-C₁₋₄-alkylcarbamoylgruppen, (13) N,N-Di-C₁₋₄-alkylcarbamoylgruppen, (14) Halogenatomen, (15) Mono-, Di- oder Trihalogen-C₁₋₄-alkylgruppen, (16) optional geschützten Aminogruppen, (17) Mono-C₁₋₄-alkylaminogruppen, (18) C₁₋₆-Alkanoylaminogruppen, (19) Benzoylaminogruppen, (20) Carbamoylaminogruppe, (21) N-C₁₋₄-Alkylcarbamoylaminogruppen, (22) N,N-Di-C₁₋₄-alkylcarbamoylaminogruppen, (23) C₁₋₃-Alkylendioxygruppen, (24) Hydroxygruppe, (25) Nitrogruppe, (26) Cyanogruppe, (27) Mercaptogruppe, (28) Sulfogruppe, (29) Sulfinogruppe, (30) Phosphonogruppe, (31) Sulfamoylgruppe, (32) C₁₋₆-Monoalkylsulfamoylgruppen, (33) Di-C₁₋₄-alkylsulfamoylgruppen, (34) C₁₋₆-Alkylthiogruppen, (35) Phenylthiogruppe, (36) C₁₋₆-Alkylsulfinylgruppen, (37) Phenylsulfinylgruppe, (38) C₁₋₆-Alkylsulfonylgruppen, (39) Phenylsulfonylgruppe und (40) einer 5- oder 6-gliedrigen heterocyclischen Gruppe, welche 1 bis 4 Heteroatome enthält, die ausgewählt sind aus Sauerstoffatom, Schwefelatom und Stickstoffatom, welche optional über eine Atomkette, die aus 1 bis 4 Kohlenstoffatomen, Sauerstoffatomen und Stickstoffatomen besteht, mit der oben erwähnten Phenylgruppe verbunden sein können, wobei ferner die oben erwähnten Gruppen mit einer Kohlenstoffkette oder einer cyclischen Gruppe, welche zwei oder mehrere Kohlenstoffatome enthalten, optional einen bis vier Substituenten besitzen können, der aus der Gruppe ausgewählt ist, bestehend aus (a) Halogenatomen, (b) Hydroxygruppe, (c) C₁₋₄-Alkoxygruppen oder Oxogruppe, (d) Di-C₁₋₄-alkylaminogruppen, (e) Halogen-C₁₋₄-Alkylgruppen, (f) C₁₋₇-Acylgruppen, (g) Hydroxy-C₁₋₄-alkylgruppen, (h) C₁₋₄-Alkoxy-C₁₋₄-alkylgruppen, (i) Sulfamoyl- und C₁₋₅-Sulfamoylgruppen, (j) Carbamoyl- und C₁₋₇-Carbamoylgruppe, (k) C₂₋₄-Alkylgruppen, (1) Carboxylgruppe, (m) C₁₋₇-Alkoxycarbonylgruppe und Phenoxycarbonyl.

3. Verfahren zur Herstellung eines 1,2,4-Triazin-3-on-Derivats nach Anspruch 2, wobei die Phenylgruppe eine Phenylgruppe ist, die an den Positionen 3 und 4 substituiert ist, oder eine Phenylgruppe ist, die an den Positionen 3, 4 und 5 substituiert ist.

4. Verfahren zur Herstellung eines 1,2,4-Triazin-3-on-Derivats nach Anspruch 1, wobei R⁴ gleich C₁₋₄-Alkyl ist.

5. Verfahren zur Herstellung eines 1,2,4-Triazin-3-on-Derivats nach Anspruch 4, wobei R⁴ gleich Methyl oder Ethyl ist.

6. Verfahren nach Anspruch 1, welches die Herstellung eines 1,2,4-Triazin-3,5-dion-Derivats umfaßt.

7. Verfahren nach Anspruch 1, welches die Herstellung eines Hexahydro-1,2,4-triazin-3-on-Derivats umfaßt.

8. Verfahren nach Anspruch 1, welches die Herstellung von 2-[4-(4-Acetylbenzyl)-3,5-dichlorphenyl]-4,5-di-hydro-1,2,4-triazin-3(2H)-on umfaßt.

9. Verfahren nach Anspruch 1, welches die Herstellung von 2-{3,5-Dichlor-4-[4-(1-hydroxyethyl)benzyl]phenyl}-4,5-dihydro-1,2,4-triazin-3(2H)-on umfaßt.

10. Verfahren nach Anspruch 1, wobei R¹ eine Gruppe der chemischen Formel: 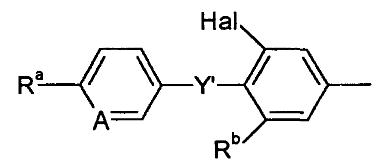 ist, wobei
R^{a} ein C₁₋₇-Acyl, ein Sulfamoyl und C₁₋₅-Sulfamoyl-gruppen, Aminocarbonyl, Carbamoyl und C₁₋₇-Carbamoylgruppen, ein C₁₋₇-Alkoxycarbonyl, ein C₂₋₄-Alkyl, Halogen-C₁₋₄-alkyl, Hydroxy-C₁₋₄-alkyl, C₁₋₄-Alkoxy-C₁₋₄-alkyl, Amino oder Di-C₁₋₄-Alkylamino ist;
A gleich -CH- oder ein Stickstoffatom ist;
Y' gleich Methylen, Cyanomethylen, Carbonyl, Hydroxymethylen, ein Schwefelatom, Sulfinyl, Sulfonyl oder ein Sauerstoffatom ist;
Hal gleich Halogen ist und
R^{b} gleich Wasserstoff, Halogen oder eine niedere-Alkylgruppe ist.

11. 1,2,4-Triazin-3-on-Derivat, das durch die chemische Formel: 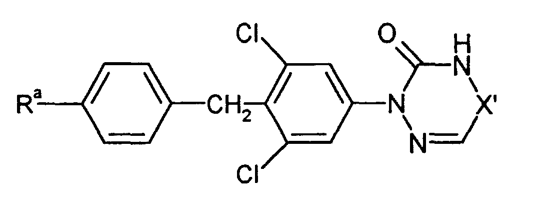 dargestellt wird, wobei R^{a} ein C₁₋₇-Acyl, ein C₁₋₅-Sulfamoyl, ein C₂₋₄-Alkyl, Halogen-C₁₋₄-alkyl, Hydroxy-C₁₋₄-alkyl oder C₁₋₄-Alkoxy-C₁₋₄-alkyl ist und X' gleich Methylen oder Carbonyl ist.

12. 1,2,4-Triazin-3-on-Derivat nach Anspruch 11, welches 2-[4-(4-Acetylbenzyl)-3,5-dichlorphenyl]-4,5-dihydro-1,2,4-triazin-3(2H)-on ist.

13. 1,2,4-Triazin-3-on-Derivat nach Anspruch 11, welches 2-(3,5-Dichlor-4-[4-(1-hydroxyethyl)-benzyl]phenyl)-4,5-dihydro-1,2,4-triazin-3(2H)-on ist.

## Revendications

1. Procédé de préparation de dérivés de 1,2,4-triazine-3-one, représentés par la formule chimique suivante : 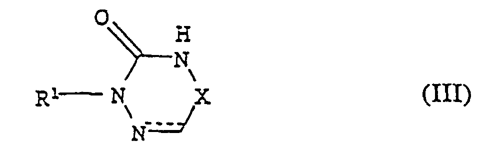 dans laquelle
R¹ représente un groupe aryle en C₆₋₁₄ ou un groupe hétérocyclique aromatique à 5 ou 6 chaînons, chacun de ces groupes pouvant éventuellement porter 1 à 5 substituants choisis dans l'ensemble constitué par
1) les groupes alkyle en C₁₋₄,
2) les groupes alcényle en C₂₋₄,
3) les groupes aralkyle en C₇₋₁₁,
4) le groupe phényle,
5) les groupes alcoxy en C₁₋₆,
6) le groupe phénoxy,
7) les groupes alcanoyle en C₁₋₆,
8) le groupe benzoyle,
9) le groupe carboxyle,
10) les groupes alcoxycarbonyle en C₂₋₇,
11) le groupe carbamyle,
12) les groupes N-mono(alkyle en C₁₋₄)carbamyle,
13) les groupes N,N-di(alkyle en C₁₋₄)carbamyle,
14) les atomes d'halogène,
15) les groupes mono-, di- ou tri-halogénoalkyle en C₁₋₄,
16) le groupe amino, éventuellement protégé,
17) les groupes mono(alkyle en C₁₋₄)amino,
18) les groupes alcanoylamino en C₁₋₆,
19) le groupe benzoylamino,
20) le groupe carbamylamino,
21) les groupes N-(alkyle en C₁₋₄)carbamylamino,
22) les groupes N,N-di(alkyle en C₁₋₄)carbamylamino,
23) les groupes alkylènedioxy en C₁₋₃,
24) le groupe hydroxyle,
25) le groupe nitro,
26) le groupe cyano,
27) le groupe mercapto,
28) le groupe sulfo,
29) le groupe sulfino,
30) le groupe phosphono,
31) le groupe sulfamyle,
32) les groupes mono(alkyle en C₁₋₆)sulfamyle,
33) les groupes di(alkyle en C₁₋₄)sulfamyle,
34) les groupes alkylthio en C₁₋₆,
35) le groupe phénylthio,
36) les groupes alkylsulfinyle en C₁₋₆,
37) le groupe phénylsulfinyle,
38) les groupes alkylsulfonyle en C₁₋₆,
39) le groupe phénylsulfonyle, et
40) les groupes hétérocycliques à 5 ou 6 chaînons, comportant 1 à 4 hétéroatomes choisis parmi les atomes d'oxygène, de soufre et d'azote, lesquels groupes peuvent être raccordés au groupe aryle en C₆₋₁₄ ou au groupe hétérocyclique aromatique à 5 ou 6 chaînons, mentionné plus haut, par une chaîne d'atomes constituée de 1 à 4 atomes de carbone, d'azote et d'oxygène,
les groupes mentionnés ci-dessus qui comportent une chaîne d'atomes de carbone ou un groupe cyclique qui contient deux atomes de carbone ou plus pouvant éventuellement porter 1 à 4 substituants choisis dans l'ensemble constitué par
a) les atomes d'halogène,
b) le groupe hydroxyle,
c) les groupes alcoxy en C₁₋₄ ou le groupe oxo,
d) les groupes di(alkyle en C₁₋₄)amino,
e) les groupes halogénoalkyle en C₁₋₄,
f) les groupes acyle en C₁₋₇,
g) les groupes hydroxyalkyle en C₁₋₄,
h) les groupes (alcoxy en C₁₋₄)alkyle en C₁₋₄,
i) les groupes sulfamyle et sulfamyle en C₁₋₅,
j) les groupes carbamyle et carbamyle en C₁₋₇,
k) les groupes alkyle en C₂₋₄,
l) le groupe carboxyle, et
m) les groupes alcoxycarbonyle en C₁₋₇ et phénoxycarbonyle ;
X représente un groupe carbonyle, un groupe thiocarbonyle ou un groupe méthylène ; et
le trait en pointillés indique qu'il peut y avoir une double liaison ;
lequel procédé comporte :
I) le fait de faire réagir un dérivé de type hydrazone, représenté par la formule chimique suivante : 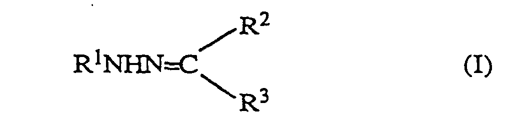 dans laquelle R¹ a la signification indiquée ci-dessus, et
R² et R³ représentent chacun
1) un atome d'hydrogène,
2) un résidu d'hydrocarbure, choisi dans l'ensemble constitué par les groupes alkyle en C₁₋₄, les groupes homocycliques aromatiques et les groupes hétérocycliques aromatiques à 5 ou 6 chaînons, ou
3) un groupe électroattracteur choisi dans l'ensemble constitué par les groupes cyano, (alcoxy en C₁₋₆)carbonyle, carboxyle, (aryloxy en C₆₋₁₀)carbonyle, les groupes (hétérocycle à 5 ou 6 chaînons)oxycarbonyle comportant 1 à 4 hétéroatomes choisis parmi les atomes d'azote, de soufre et d'oxygène, les groupes alkylsulfonyle en C₁₋₆, qui peuvent éventuellement porter 1 à 3 substituants halogéno, le groupe aminosulfonyle, les groupes di(alcoxy en C₁₋₄)phosphoryle, les groupes acyle en C₁₋₆, qui peuvent éventuellement porter 1 à 3 substituants halogéno, le groupe carbamyle et les groupes (alkyle en C₁₋₆)sulfonylthiocarbamyle,
avec un isocyanate de dialcoxyéthyle, représenté par la formule
(R⁴O)₂CHCH₂NCO
dans laquelle R⁴ représente un groupe alkyle en C₁₋₄, les deux groupes symbolisés par R⁴ pouvant aussi former l'un avec l'autre un cycle à chaîne de type alkylène, pour obtenir un dérivé de type semicarbazone, représenté par la formule chimique suivante : 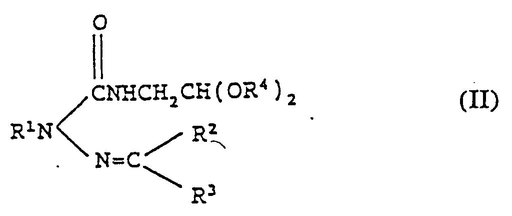 dans laquelle les divers symboles ont les significations indiquées ci-dessus,
et puis
II) le fait de soumettre ce dérivé de type semicarbazone à une réaction de cyclisation.

2. Procédé de préparation de dérivés de 1,2,4-triazine-3-one, conforme à la revendication 1, dans lequel R¹ représente un groupe phényle qui peut éventuellement porter 1 à 5 substituants choisis dans l'ensemble constitué par
1) les groupes alkyle en C₁₋₄,
2) les groupes alcényle en C₂₋₄,
3) les groupes aralkyle en C₇₋₁₁,
4) le groupe phényle,
5) les groupes alcoxy en C₁₋₆,
6) le groupe phénoxy,
7) les groupes alcanoyle en C₁₋₆,
8) le groupe benzoyle,
9) le groupe carboxyle,
10) les groupes alcoxycarbonyle en C₂₋₇,
11) le groupe carbamyle,
12) les groupes N-mono(alkyle en C₁₋₄)carbamyle,
13) les groupes N,N-di(alkyle en C₁₋₄)carbamyle,
14) les atomes d'halogène,
15) les groupes mono-, di- ou tri-halogénoalkyle en C₁₋₄,
16) le groupe amino, éventuellement protégé,
17) les groupes mono(alkyle en C₁₋₄)amino,
18) les groupes alcanoylamino en C₁₋₆,
19) le groupe benzoylamino,
20) le groupe carbamylamino,
21) les groupes N-(alkyle en C₁₋₄)carbamylamino,
22) les groupes N,N-di(alkyle en C₁₋₄)carbamylamino,
23) les groupes alkylènedioxy en C₁₋₃,
24) le groupe hydroxyle,
25) le groupe nitro,
26) le groupe cyano,
27) le groupe mercapto,
28) le groupe sulfo,
29) le groupe sulfino,
30) le groupe phosphono,
31) le groupe sulfamyle,
32) les groupes mono(alkyle en C₁₋₆)sulfamyle,
33) les groupes di(alkyle en C₁₋₄)sulfamyle,
34) les groupes alkylthio en C₁₋₆,
35) le groupe phénylthio,
36) les groupes alkylsulfinyle en C₁₋₆,
37) le groupe phénylsulfinyle,
38) les groupes alkylsulfonyle en C₁₋₆,
39) le groupe phénylsulfonyle, et
40) les groupes hétérocycliques à 5 ou 6 chaînons, comportant 1 à 4 hétéroatomes choisis parmi les atomes d'oxygène, de soufre et d'azote, lesquels groupes peuvent être raccordés au groupe phényle mentionné plus hau, par une chaîne d'atomes constituée de 1 à 4 atomes de carbone, d'azote et d'oxygène,
les groupes mentionnés ci-dessus qui comportent une chaîne d'atomes de carbone ou un groupe cyclique qui contient deux atomes de carbone ou plus pouvant éventuellement porter 1 à 4 substituants choisis dans l'ensemble constitué par
a) les atomes d'halogène,
b) le groupe hydroxyle,
c) les groupes alcoxy en C₁₋₄ ou le groupe oxo,
d) les groupes di(alkyle en C₁₋₄)amino,
e) les groupes halogénoalkyle en C₁₋₄,
f) les groupes acyle en C₁₋₇,
g) les groupes hydroxyalkyle en C₁₋₄,
h) les groupes (alcoxy en C₁₋₄)alkyle en C₁₋₄,
i) les groupes sulfamyle et sulfamyle en C₁₋₅,
j) les groupes carbamyle et carbamyle en C₁₋₇,
k) les groupes alkyle en C₂₋₄,
l) le groupe carboxyle, et
m) les groupes alcoxycarbonyle en C₁₋₇ et phénoxycarbonyle.

3. Procédé de préparation de dérivés de 1,2,4-triazine-3-one, conforme à la revendication 2, dans lequel le groupe phényle est un groupe phényle portant des substituants en positions 3 et 4, ou un groupe phényle portant des substituants en positions 3, 4 et 5.

4. Procédé de préparation de dérivés de 1,2,4-triazine-3-one, conforme à la revendication 1, dans lequel R⁴ représente un groupe alkyle en C₁₋₄.

5. Procédé de préparation de dérivés de 1,2,4-triazine-3-one, conforme à la revendication 4, dans lequel R⁴ représente un groupe méthyle ou éthyle.

6. Procédé conforme à la revendication 1, qui consiste à préparer un dérivé de 1,2,4-triazine-3,5-dione.

7. Procédé conforme à la revendication 1, qui consiste à préparer un dérivé d'hexahydro-1,2,4-triazine-3-one.

8. Procédé conforme à la revendication 1, qui consiste à préparer de la 2-[4-(4-acétylbenzyl)-3,5-dichlorophényl]-4,5-dihydro-1,2,4-triazine-3 (2H)-one.

9. Procédé conforme à la revendication 1, qui consiste à préparer de la 2-{3,5-dichloro-4-[4-(1-hydroxyéthyl)benzyl]phényl}-4,5-dihydro-1,2,4-triazine-3(2H)-one.

10. Procédé conforme à la revendication 1, dans lequel R¹ représente un groupe de formule chimique suivante : 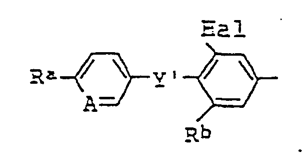 dans laquelle
R^{a} représente un groupe acyle en C₁₋₇, sulfamyle et sulfamyle en C₁₋₅, aminocarbonyle, carbamyle et carbamyle en C₁₋₇, alcoxycarbonyle en C₁₋₇, alkyle en C₂₋₄, halogénoalkyle en C₁₋₄, hydroxyalkyle en C₁₋₄, (alcoxy en C₁₋₄)alkyle en C₁₋₄, amino ou di(alkyle en C₁₋₄)amino, A représente un chaînon -CH- ou un atome d'azote,
Y' représente un groupe méthylène, cyanométhylène, hydroxyméthylène, carbonyle, sulfinyle ou sulfonyle, ou un atome de soufre ou d'oxygène,
Hal représente un atome d'halogène, et
R^{b} représente un atome d'hydrogène ou d'halogène ou un groupe alkyle inférieur.

11. Dérivé de 1,2,4-triazine-3-one représenté par la formule chimique suivante : 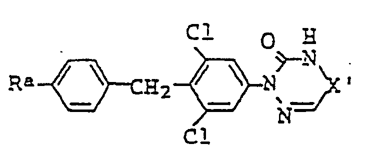 dans laquelle
R^{a} représente un groupe acyle en C₁₋₇, sulfamyle et sulfamyle en C₁₋₅, alkyle en C₂₋₄, halogénoalkyle en C₁₋₄, hydroxyalkyle en C₁₋₄, ou (alcoxy en C₁₋₄)alkyle en C₁₋₄, et
X' représente un groupe méthylène ou carbonyle.

12. Dérivé de 1,2,4-triazine-3-one conforme à la revendication 11, qui est la 2-[4-(4-acétylbenzyl)-3,5-dichlorophényl]-4,5-dihydro-1,2,4-triazine-3(2H)-one.

13. Dérivé de 1,2,4-triazine-3-one conforme à la revendication 11, qui est la 2-{3,5-dichloro-4-[4-(1-hydroxyéthyl)benzyl]phényl}-4,5-dihydro-1,2,4-triazine-3 (2H)-one.
